(19) Europäisches Patentamt
European Patent Office
Office européen des brevets

(11) EP 1 349 533 B1

(12) # EUROPÄISCHE PATENTSCHRIFT

(45) Veröffentlichungstag und Bekanntmachung des Hinweises auf die Patenterteilung:
**30.08.2006 Patentblatt 2006/35**

(21) Anmeldenummer: **01997280.1**

(22) Anmeldetag: **15.11.2001**

(51) Int Cl.:
*A61K 8/04* (2006.01) *A61K 8/06* (2006.01)
*A61K 8/26* (2006.01) *A61Q 15/00* (2006.01)
*A61Q 19/00* (2006.01) *A61Q 19/08* (2006.01)
*A61K 9/12* (2006.01) *A61K 9/107* (2006.01)

(86) Internationale Anmeldenummer:
**PCT/EP2001/013223**

(87) Internationale Veröffentlichungsnummer:
**WO 2002/041848 (30.05.2002 Gazette 2002/22)**

(54) **TREIBGASFREIE SPRAYZUBEREITUNGEN**

PROPELLANT-GAS-FREE SPRAY PREPARATIONS

PREPARATIONS POUR PULVERISATION EXEMPTES DE GAZ PROPULSEUR

(84) Benannte Vertragsstaaten:
**AT BE CH CY DE DK ES FI FR GB GR IE IT LI LU MC NL PT SE TR**

(30) Priorität: **23.11.2000 DE 10058224**

(43) Veröffentlichungstag der Anmeldung:
**08.10.2003 Patentblatt 2003/41**

(73) Patentinhaber: **Henkel Kommanditgesellschaft auf Aktien**
**40589 Düsseldorf (DE)**

(72) Erfinder:
- **BANOWSKI, Bernhard**
  **40597 Düsseldorf (DE)**
- **PÖPPL, Marion**
  **41564 Kaarst (DE)**

(56) Entgegenhaltungen:
**EP-A- 0 733 357** **WO-A-00/66076**
**DE-A- 2 519 860** **DE-A- 4 009 347**
**DE-A- 10 008 839** **DE-A- 19 530 220**
**DE-A1- 19 514 269** **US-A- 4 338 294**

- **FLOYD, DAVID T.: "Rheological agents for antiperspirants" COSMET. TOILETRIES (1981), 96(1), 21-7 , XP002202512**
- **PATENT ABSTRACTS OF JAPAN vol. 1998, no. 01, 30. Januar 1998 (1998-01-30) & JP 09 241152 A (SUNSTAR INC), 16. September 1997 (1997-09-16)**

EP 1 349 533 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft Sprayzubereitungen zur topischen Applikation am menschlichen Körper, bestehend aus einem Spendebehälter mit Sprühapplikator und einer flüssigen Wirkstoffzubereitung in Form einer feinteiligen Öl-in-Wasser-Emulsion oder Öl-in-Wasser-Mikroemulsion, die sich aufgrund ihrer Zusammensetzung und Rheologie durch besonders günstige Anwendungseigenschaften auszeichnet.

Für die feine Verteilung von wirkstoffhaltigen Zubereitungen auf dem Körper oder den Haaren haben Aerosolsprays wegen ihrer bequemen Handhabung und der feinen Verteilung, die damit erreicht wird, eine besondere Beliebtheit erlangt. Aus Gründen der ökologischen Bedenklichkeit der Fluorkohlenwasserstoff Treibgase und der Entflammbarkeit von Kohlenwasserstoff-Treibgasen und Dimethylether haben auch treibgasfreie Produkte, z. B. Pumpzerstäuber, Bedeutung am Markt.
Solche Systeme haben bisher in Form der altbekannten Ballonpumpen für die Parfümzerstäubung, der flexiblen "Squeeze-Bottle" und der starren Sprühflaschen mit Finger-oder Handhebelpumpen praktische Anwendungen gefunden. Auch elektrisch betriebene Pumpzerstäuber sind bekannt.
Zu den treibgasfreien Spraysystemen zählen darüber hinaus noch die Elastomer-Drucksprühsysteme, in denen ein Elastomer-Beutel in einem starren Gehäuse mit einem Sprühventil in Verbindung steht und mit einem flüssigen Produkt unter Druck abgefüllt ist. Dabei ist die Sprühdruck-Energie in dem Elastomer-Beutel gespeichert.
Die größte Bedeutung haben bisher die Pump-Sprühflaschen mit Finger oder Handpumpen erlangt.

Ein Problem, das bei den meisten aerosolfreien Sprühspendem besteht, ist die Ausbringung eines ausreichend feinen Sprühstrahls, der die versprühte Flüssigkeit fein zerteilt und in dünner Schicht auf die Körper- oder Hautoberfläche aufbringt, ohne daß die ausgebrachte Flüssigkeit von der Haut oder dem Haar herunter tropft oder auf der Haut ein unangenehmes Nässegefühl erzeugt.

Herkömmliche Produkte, insbesondere solche auf wäßriger und emulsionsförmiger Basis, zeigen, wenn sie in ausreichender Menge aufgetragen werden, ein deutliches, tropfenförmiges Ablaufen von der Hautoberfläche.

Die Erfinder haben sich die Aufgabe gestellt, die Versprühbarkeit von treibgasfreien Sprayzubereitungen und die Abscheidung der versprühten Zubereitungen auf der Haut durch die Formulierung der Zubereitung zu verbessern. Sie haben dabei die Beobachtung gemacht, daß das Verhalten von Sprays auf Basis einer feinteiligen, wäßrigen Emulsion oder Mikroemulsion auf der Haut durch die Rheologie der Formulierungen erheblich verbessert werden kann. Der Einsatz polymerer organischer Verdickungsmittel führt in emulsionsförmigen Zubereitungen zu Unverträglichkeiten mit den enthaltenen Komponenten und zeigt nicht die erwünschten rheologischen Eigenschaften.

Die Verwendung von Schichtsilikaten ist in schweißhemmenden, wäßrig-alkoholischen treibgashaltigen Aerosolzubereitungen aus DE 25 19 860 A1, in wasserfreien Sprayzubereitungen aus DE 40 09 347 A1 bekannt.

Gegenstand der Erfindung ist eine treibgasfreie Sprayzubereitung zur topischen Applikation am menschlichen Körper, bestehend aus einem Spendebehälter mit Sprühapplikator und einer flüssigen Wirkstoffzubereitung in Form einer feinteiligen, strukturviskosen Öl-in-Wasser-Emulsion oder Öl-in-Wasser-Mikroemulsion mit einer Tröpfchengröße unter 500 nm (Nanometer), enthaltend

(A) eine feinteilige Öl-in-Wasser-Emulsion oder Öl-in-Wasser-Mikroemulsion mit einem Anteil von 0,5-30 Gew.-% emulgierter Lipide und 0,2-10 Gew.-% Emulgatoren, jeweils bezogen auf die gesamte flüssige Wirkstoffzubereitung,

(B) 1-30 Gew.-%, bezogen auf die gesamte flüssige Wirkstoffzubereitung, wenigstens eines kosmetischen oder dermatologischen Wirkstoffs,

(C) 0,2-2 Gew.%, bezogen auf die gesamte flüssige Wirkstoffzubereitung, eines verdickenden Schichtsilikats,

sowie gegebenenfalls bis zu 15 Gew.-%, bezogen auf die gesamte flüssige Wirkstoffzubereitung, weiterer Hilfsmittel.

Durch den Anteil des verdickenden Schichtsilikats bildet sich in dem Produkt eine hohe Strukturviskosität aus, d. h. daß die scheinbare Viskosität unter dem Einfluß zunehmender Scherbelastung spontan stark abnimmt und nach Wegfall der Scherbelastung spontan wieder ansteigt. Bei einer Scherbelastung von D = 1/s sollte die scheinbare Viskosität bei 25 °C oberhalb von 100 m·Pa·s liegen, bei einer Scherbeiastung von 100/s und mehr sollte die scheinbare Viskosität nicht mehr als 50 %, bevorzugt nicht mehr als 20 % und besonders bevorzugt nicht mehr als 5 % der Viskosität bei 1/s betragen (Beschreibung des Messverfahrens siehe unten).
Unter die Definition "strukturviskos" fallen erfindungsgemäß auch thixotrope Öl-in-Wasser-Emulsionen oder Öl-in-Wasser-Mikroemulsionen, deren Viskositätsabfall unter Scherbelastung mit einer Zeitverzögerung von maximal 1 Sekunde auftritt und deren Viskositätsanstieg nach Wegfall der Scherbelastung innerhalb von maximal 2 Sekunden, bevorzugt innerhalb von maximal 1 Sekunde erfolgt. Die Viskosität nach dem Wegfall der Scherbelastung muss dabei nicht auf den ursprünglichen Wert ansteigen; es genügt, wenn die Viskosität so hoch ist, dass die Emulsion nicht vom besprühten Areal herabtropft.

Durch die Strukturviskosität oder die Thixotropie mit geringer Zeitverzögerung wird erreicht, daß sich die Emulsion unter der Scherkraft des Sprühvorgangs fein zerstäuben läßt, aber auf der Hautoberfläche spontan oder nur mit sehr kurzer Zeitverzögerung wieder verdickt, so daß sie nicht herabtropft. Die Emulsion oder Mikroemulsion wird auf dem besprühten Areal fixiert und der Wirkstoff optimal genutzt.
Bevorzugt sind hierbei die strukturviskosen Zusammensetzungen, deren Viskosität zeitunabhängig von der Scherbelastung abhängt.

Die erfindungsgemäßen Sprayzubereitungen können in jedem beliebigen treibgasfreien Spraysystem, das einen Spendebehälter und einen Sprühapplikator aufweist, enthalten sein, also z. B. in einer flexiblen Druckflasche mit Tauchrohr und Sprühventil (Squeeze Bottle), in einem Ballonzerstäuber, der nach dem Venturiprinzip arbeitet oder in einer Pumpen-Sprühflasche, deren Pumpe mit dem Finger oder mit der Hand, z. B. in der Art eines Abzugsbügels betätigt wird. In einer für die kosmetische Anwendung bevorzugten Ausführung weist der Spendebehälter eine manuell betätigte Sprühpumpe auf.

Die erfindungsgemäße treibgasfreie Sprayzubereitung eignet sich zur Verteilung flüssiger Wirkstoffzubereitungen auf festen Oberflächen, bevorzugt auf der Haut oder dem Haar. Als Wirkstoffe können alle bekannten Stoffe mit einer kosmetischen oder dermatologischen Wirkung eingesetzt werden, die sich in Wasser oder in dem Lipid lösen oder feinteilig emulgieren oder dispergieren lassen.

Ein weiterer Gegenstand der vorliegenden Erfindung ist ein Verfahren zur Herstellung einer treibgasfreien Sprayzubereitung zur topischen Applikation am menschlichen Körper, die aus einem Spendebehälter mit Sprühapplikator und einer flüssigen Wirkstoffzubereitung in Form einer feinteiligen, strukturviskosen Öl-in-Wasser-Emulsion oder Öl-in-Wasser-Mikroemulsion mit einer Tröpfchengröße unter 500 nm besteht, wobei eine feinteilige Öl-in-Wasser-Emulsion oder Öl-in-Wasser-Mikroemulsion mit einem Anteil von 0,5 - 30 Gew.-% emulgierter Lipide, 0,2 - 10 Gew.-% Emulgatoren, 1-30 Gew.-% wenigstens eines kosmetischen oder dermatologischen Wirkstoffs, 0,2 - 2 Gew.-% eines verdickenden Schichtsilikats, gegebenenfalls bis zu 15 Gew.-% weiterer Hilfsmittel, jeweils bezogen auf die gesamte flüssige Wirkstoffzubereitung, hergestellt und in einen treibgasfreien Spendebehälter mit Sprühapplikator eingefüllt wird.

Ein weiterer Gegenstand der vorliegenden Erfindung ist die Verwendung von mindestens einem verdickenden Schichtsilikat zur Verbesserung der Applikationseigenschaften einer treibgasfreien Sprayzubereitung zur topischen Applikation am menschlichen Körper, die aus einem Spendebehälter mit Sprühapplikator und einer flüssigen Wirkstoffzubereitung in Form einer feinteiligen, strukturviskosen Öl-in-Wasser-Emulsion oder Öl-in-Wasser-Mikroemulsion mit einer Tröpfchengröße unter 500 nm besteht und eine feinteilige Öl-in-Wasser-Emulsion oder Öl-in-Wasser-Mikroemulsion mit einem Anteil von 0,5 - 30 Gew.-% emulgierter Lipide, 0,2 - 10 Gew.-% Emulgatoren, 1 - 30 Gew.-% wenigstens eines kosmetischen oder dermatologischen Wirkstoffs, 0,2 - 2 Gew.-% eines verdickenden Schichtsilikats sowie gegebenenfalls bis zu 15 Gew.-% weiterer Hilfsmittel, jeweils bezogen auf die gesamte flüssige Wirkstoffzubereitung, enthält.

Als kosmetische oder dermatologische Wirkstoffe erfindungsgemäß geeignet sind zum Beispiel Antitranspirantien. Als Antitranspirant-Wirkstoffe eignen sich wasserlösliche adstringierende oder einweißkoagulierende metallische Salze, insbesondere anorganische und organische Salze des Aluminiums, Zirkoniums, Zinks und Titans sowie beliebige Mischungen dieser Salze. Erfindungsgemäß wird unter Wasserlöslichkeit eine Löslichkeit von wenigstens 5 g Aktivsubstanz pro 100 g Lösung bei 20 °C verstanden. Erfindungsgemäß verwendbar sind beispielsweise Alaun ($KAl(SO_4)_2 \cdot 12\ H_2O$), Aluminiumsulfat, Aluminiumlactat, Natrium-Aluminium-Chlorhydroxylactat, Aluminiumchlorhydroxyallantoinat, Aluminiumchlorohydrat, Aluminiumsulfocarbolat, Aluminium-ZirkoniumChlorohydrat, Zinkchlorid, Zinksulfocarbolat, Zinksulfat, Zirkoniumchlorohydrat, Aluminium-Zirkonium-Chlorohydrat-Glycin-Komplexe und Komplexe von basischen Aluminiumchloriden mit Propylenglycol oder Polyethylenglycol. Bevorzugt enthalten die flüssigen Wirkstoffzubereitungen ein adstringierendes Aluminiumsalz, insbesondere Aluminiumchlorohydrat, und/ oder eine Aluminium-Zirkonium-Verbindung. Sie sind in den erfindungsgemäßen Wirkstoffzubereitungen in einer Menge von 1 - 40 Gew.-%, vorzugsweise 2 - 30 Gew.-% und insbesondere 5 - 25 Gew.-% enthalten (bezogen auf die Menge der Aktivsubstanz in der gesamten flüssigen Wirkstoffzubereitung). Aluminiumchlorohydrate werden beispielsweise pulverförmig als Micro Dry® Ultrafine oder in aktivierter Form als Reach® 501 von Reheis sowie in Form wäßriger Lösungen als Locron® L von Clariant oder als Chlorhydrol® von Reheis vertrieben. Unter der Bezeichnung Reach® 301 wird ein Aluminiumsesquichlorohydrat von Reheis angeboten. Auch die Verwendung von Aluminium-Zirkonium-Tetrachlorohydrex-Glycin-Komplexen, die beispielsweise von Reheis unter der Bezeichnung Rezal® 36G im Handel sind, ist erfindungsgemäß besonders vorteilhaft.
Besonders gut eignen sich die erfindungsgemäßen Sprayzubereitungen zur Applikation von schweißhemmenden, adstringierenden Salzen, da solche Zubereitungen z. B. mit wasserlöslichen polymeren Verdickungsmitteln wenig verträglich sind und zur Instabilität führen.

Ebenfalls als kosmetische oder dermatologische Wirkstoffe erfindungsgemäß geeignet sind Deodorantien. Als Deodorantien können Duftstoffe, antimikrobielle, antibakterielle oder keimhemmende Stoffe, enzymhemmende Stoffe, Antioxidantien oder Geruchsadsorbentien eingesetzt werden.
Geeignete antimikrobielle, antibakterielle oder keimhemmende Stoffe sind insbesondere Organohalogenverbindungen sowie -halogenide, quartäre Ammoniumverbindungen, eine Reihe von Pflanzenextrakten und Zinkverbindungen. Bevorzugt sind halogenierte Phenolderivate wie z. B. Hexachlorophen oder Irgasan DP 300 (Triclosan, 2,4,4'-Trichlor-2'-hydroxydiphenylether), 3,4,4'-Trichlorcarbonilid, Chlorhexidin (1,1'-Hexamethylen-bis-[5-(4-chlorphenyl)]-biguanid), Chlorhexidingluconat, Benzalkoniumhalogenide und Cetylpyridiniumchlorid. Desweiteren sind Natriumbicarbonat, Natriumphenolsulfonat und Zinkphenolsulfonat sowie z. B. die Bestandteile des Lindenblütenöls einsetzbar. Auch schwächer wirksame antimikrobielle Stoffe, die aber eine spezifische Wirkung gegen die für die Schweißzersetzung verantwortlichen grampositiven Keime haben, können als Deodorant-Wirkstoffe eingesetzt werden. Zu diesen zählen viele ätherische Öle wie z. B. Nelkenöl (Eugenol), Minzöl (Menthol) oder Thymianöl (Thymol) sowie Terpenalkohole wie z. B. Farnesol. Auch aromatische Alkohole wie z. B. Benzylalkohol, 2-Phenylethanol oder 2-Phenoxyethanol können als Deodorant-Wirkstoffe eingesetzt werden. Weitere antibakteriell wirksame Deodorantien sind Lantibiotika, Glycoglycerolipide, Sphingolipide (Ceramide), Sterine und andere Wirkstoffe, die die Bakterienadhäsion an der Haut inhibieren, z. B. Glycosidasen, Lipasen, Proteasen, Kohlenhydrate, Di- und Oligosaccharidfettsäureester sowie alkylierte Mono- und Oligosaccharide. Ebenfalls geeignet sind langkettige Diole, z. B. 1,2-Alkan-($C_8$-$C_{18}$)-Diole, Glycerinmono-($C_6$-$C_{16}$)-alkylether oder Glycerinmono($C_8$-$C_{18}$)-Fettsäureester, die sehr gut haut- und schleimhautverträglich und gegen Corynebakterien wirksam sind.

Als enzymhemmende Stoffe sind vor allem solche deodorierend wirksam, die esterspaltende Enzyme inhibieren und auf diese Weise der Schweißzersetzung entgegenwirken. Hierfür eignen sich vor allem die Ester von aliphatischen $C_2$-$C_6$-Carbonsäuren oder Hydroxycarbonsäuren und $C_2$-$C_6$-Alkoholen oder Polyolen, z. B. Triethylcitrat, Propylenglycollactat oder Glycerintriacetat (Triacetin).

Antioxidative Stoffe können der oxidativen Zersetzung der Schweißkomponenten entgegenwirken und auf diese Weise die Geruchsentwicklung hemmen. Geeignete Antioxidantien sind Aminosäuren (z. B. Glycin, Histidin, Tyrosin, Tryptophan) und deren Derivate, Imidazol und Imidazolderivate (z. B. Urocaninsäure), Peptide wie z. B. D,L-Carnosin, D-Carnosin, L-Carnosin und deren Derivate (z. B. Anserin), Carotinoide, Carotine (z. B. α-Carotin, β-Carotin, Lycopin) und deren Derivate, Liponsäure und deren Derivate (z. B. Dihydroliponsäure), Aurothioglucose, Propylthiouracil und weitere Thioverbindungen (z. B. Thioglycerin, Thiosorbitol, Thioglycolsäure, Thioredoxin, Glutathion, Cystein, Cystin, Cystamin und deren Glycosyl-, N-Acetyl-, Methyl-, Ethyl-, Propyl-, Amyl-, Butyl-, Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol/kg bis pmol/kg), ferner Metallchelatoren (z. B. α-Hydroxyfettsäuren, EDTA, EGTA, Phytinsäure, Lactoferrin), α-Hydroxysäuren (z. B. Zitronensäure, Milchsäure, Äpfelsäure), Huminsäuren, Gallensäure, Gallenextrakte, Gallussäureester (z. B. Propyl-, Octyl- und Dodecylgallat), Flavonoide, Catechine, Bilirubin, Biliverdin und deren Derivate, ungesättigte Fettsäuren und deren Derivate (z. B. γ-Linolensäure, Linolsäure, Arachidonsäure, Ölsäure), Folsäure und deren Derivate, Hydrochinon und dessen Derivate (z. B. Arbutin), Ubichinon und Ubichinol sowie deren Derivate, Vitamin C und dessen Derivate (z. B. Ascorbylpalmitat, -stearat, -dipalmitat, -acetat, Mg-Ascorbylphosphate, Natrium- und Magnesiumascorbat, Dinatriumascorbylphosphat und - sulfat, Kaliumascorbyltocopherylphosphat, Chitosanascorbat), Isoascorbinsäure und deren Derivate, Tocopherole und deren Derivate (z. B. Tocopherylacetat,-linoleat, -oleat und -succinat, Tocophereth-5, Tocophereth-10, Tocophereth-12, Tocophereth-18, Tocophereth-50, Tocophersolan), Vitamin A und Derivate (z. B. Vitamin-A-Palmitat), das Coniferylbenzoat des Benzoeharzes, Rutin, Rutinsäure und deren Derivate, Dinatriumrutinyldisulfat, Zimtsäure und deren Derivate (z. B. Ferulasäure, Ethylferulat, Kaffeesäure), Kojisäure, Chitosanglycolat und -salicylat, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und Zink-Derivate (z. B. ZnO, $ZnSO_4$), Selen und Selen-Derivate (z. B. Selenmethionin), Stilbene und Stilben-Derivate (z. B. Stilbenoxid, trans-Stilbenoxid). Erfindungsgemäß können geeignete Derivate (Salze, Ester, Zucker, Nukleotide, Nukleoside, Peptide und Lipide) sowie Mischungen dieser genannten Wirkstoffe oder Pflanzenextrakte (z. B. Teebaumöl, Rosmarinextrakt und Rosmarinsäure), die diese Antioxidantien enthalten, eingesetzt werden.

Als lipophile, öllösliche Antioxidantien aus dieser Gruppe sind Tocopherol und dessen Derivate, Gallussäureester, Flavonoide und Carotinoide sowie Butylhydroxytoluol/anisol bevorzugt. Als wasserlösliche Antioxidantien sind Aminosäuren, z. B. Tyrosin und Cystein und deren Derivate sowie Gerbstoffe, insbesondere solche pflanzlichen Ursprungs bevorzugt.

Die Gesamtmenge der Antioxidantien in den erfindungsgemäßen flüssigen Wirkstoffzubereitungen beträgt 0,001 - 10 Gew.-%, vorzugsweise 0,05 - 5 Gew.-% und insbesondere 0,05 - 2 Gew.-%, bezogen auf die gesamte Wirkstoffzubereitung.

Als Geruchsabsorber können folgende Substanzen eingesetzt werden: Zinkricinoleat, Cyclodextrin und dessen Derivate, Hydroxypropyt-β-Cydodextrin, weiterhin Oxide wie Magnesiumoxid oder Zinkoxid, wobei die Oxide nicht mit Aluminiumchlorhydrat kompatibel sind, weiterhin Stärke und Stärkederivate, Kieselsäuren, die ggf. modifiziert sein können, Zeolithe, Talcum sowie synthetische Polymere, z.B. Nylon.

Auch komplexbildende Stoffe können die deodorierende Wirkung unterstützen, indem sie die oxidativ katalytisch wirkenden Schwermetallionen (z. B. Eisen oder Kupfer) stabil komplexieren. Geeignete Komplexbildner sind z. B. die Salze der Ethylendiamintetraessigsäure oder der Nitrilotriessigsäure sowie die Salze der 1-Hydroxyethan-1,1-diphosphonsäure.
Als Duftstoffe oder Parfümöle können einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 C-Atomen, Citral, Citronellal, Citronellyloxyacetaldehyd, Cyclamenaldehyd, Hydroxycitronellal, Lilial und Bourgeonal, zu den Ketonen, z.B. die Ionone ∝-Isomethylionen und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen.
Geeignete Parfümöle können auch natürliche Riechstoffgemische enthalten, wie sie aus pflanzlichen oder tierischen Quellen zugänglich sind, z.B. Pinien-, Citrus-, Jasmin-, Lilien-, Rosen- oder Ylang-Ylang-Öl. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Laudanumöl.

Weitere Wirkstoffe, die sich mit den erfindungsgemäßen Wirkstoffzubereitungen formulieren lassen, sind organische und/oder anorganische UV-Filtersubstanzen.
Erfindungsgemäß geeignete organische UV-Filter sind ausgewählt aus den Derivaten von Dibenzoylmethan, Zimtsäureestern, Diphenylacrylsäureestem, Benzophenon, Campher, p-Aminobenzoesäureestern, o-Aminobenzoesäureestern, Salicylsäureestem, Benzimidazolen, 1,3,5-Triazinen, monomeren und oligomeren 4,4-Diarylbutadiencarbonsäureestern und -carbonsäureamiden, Ketotricyclo(5.2.1.0)decan, Benzalmalonsäureestern sowie beliebigen Mischungen der genannten Komponenten. Die organischen UV-Filter können öllöslich oder wasserlöslich sein.
Erfindungsgemäß besonders bevorzugte öllösliche UV-Filter sind 1-(4-tert.-Butylphenyl)-3-(4'methoxyphenyl)propan-1,3-dion (z. B. Parsol® 1789), 1-Phenyl-3-(4'-isopropylphenyl)-propan-1,3-dion, 3-(4'-Methylbenzyliden)-D,L-campher, 4-(Dimethylamino)-benzoesäure-2-ethylhexylester, 4-(Dimethylamino)benzoesäure-2-octylester, 4-(Dimethyl-amino)-benzoesäureamylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethylhexylester (Octocrylene), Salicylsäure-2-ethylhexylester, Salicylsäure-4-isopropylbenzylester, Salicylsäurehomomenthylester (3,3,5-Trimethyl-cyclohexylsalicylat), 2-Hydroxy-4-methoxybenzophenon, 2-Hydroxy-4-methoxy-4'-methylbenzophenon, 2,2'-Dihydroxy-4-methoxybenzophenon, 4-Methoxybenzmalonsäuredi-2-ethylhexylester, 2,4,6-Trianilino-(p-carbo-2'-ethyl-1'-hexyloxy)-1,3,5-triazin (Octyl Triazone) und Dioctyl Butamido Triazone (Uvasorb® HEB) sowie beliebige Mischungen der genannten Komponenten.
Bevorzugte wasserlösliche UV-Filter sind 2-Phenylbenzimidazol-5-sulfonsäure und deren Alkali-, Erdalkali-, Ammonium-, Alkylammonium-, Alkanolammonium- und Glucammoniumsalze, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z. B. 4-(2-Oxo-3-bomylidenmethyl) benzolsulfonsäure und 2-Methyl-5-(2-oxo-3-bomyliden)sulfonsäure und deren Salze.
Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'methoxyphenyl)propan-1,3-dion (z. B. Parsol® 1789) in verschiedenen UV-B-Filtern herstellen. Die erfindungsgemäßen Wirkstoffzubereitungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexylsalicylat. In diesen Kombinationen liegt das Gewichtsverhältnis von UV-B-Filter zu 1-(4-tert.-Butylphenyl)-3-(4'methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0.
Erfindungsgemäß geeignete anorganische Lichtschutzpigmente sind ausgewählt aus feindispersen Metalloxiden und Metallsalzen, insbesondere Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die bevorzugten anorganischen Partikelsubstanzen sind hydrophil oder amphiphil. Vorteilhafterweise können sie oberflächlich beschichtet, d.h. hydrophilisiert oder hydrophobiert vorliegen. Bevorzugt sind die Pigmente oberflächlich wasserabweisend behandelt ("gecoatet"). Typische Beispiele sind gecoatete Titandioxide, wie z. B. Titandioxid T 805 (Degussa), oder mit Aluminiumstearat beschichtetes Titandioxid (Handelsprodukt MT 100 T von der Firma Tayca). Als hydrophobe Coatingmittel bevorzugt sind Silicone und dabei speziell Trialkoxyoctylsilane oder Simethicone. Bevorzugte Pigmente dieses Typs sind z. B. mit Dimethylpolysiloxan (Dimethicone) beschichtetes Zinkoxid, mit Dimethicone beschichtetes Bomitrid (Très BN® UHP 1106 von Carborundum),

mit einem Gemisch aus Dimethylpolysiloxan und Silicagel (Simethicone) und Aluminiumoxidhydrat (Alumina) beschichtetes Titandioxid (Eusolex® T 2000 von Merck), mit Octylsilanol beschichtetes Titandioxid oder sphärische Polyalkylsesquioxan-Partikel (Aerosil® R972 und Aerosil® 200V von Degussa). Die Lichtschutzpigmente können dabei sowohl in Pulverform als auch in vordispergierter Form in die erfindungsgemäßen Emulsionen oder Mikroemulsionen beziehungsweise in deren Öl- oder Wasserphase eingearbeitet werden. Auch die Vordispergierung kann sowohl in einer öl- als auch in einer wasserhaltigen Phase erfolgen.
Besonders bevorzugte anorganische Lichtschutzpigmente sind ausgewählt aus den Oxiden von Titan, Zink, Cer und Eisen sowie beliebigen Mischungen der genannten Komponenten, die oberflächenmodifiziert sein können.

Desweiteren lassen sich die erfindungsgemäßen Wirkstoffzubereitungen mit insektenabweisenden Wirkstoffen, z. B. N,N-Diethyl-3-methylbenzamid (DEET), formulieren.

Desweiteren lassen sich die erfindungsgemäßen Wirkstoffzubereitungen mit weiteren kosmetischen Wirkstoffen, z. B. Vitaminen, Proteinhydrolysaten oder Pflanzenextrakten, formulieren. Viele dieser Substanzen zeigen eine Antifalten- oder Anti-Age-Wirkung, so dass die entsprechenden erfindungsgemäßen Sprayzubereitungen als Antifalten- oder Anti-Age-Mittel verwendet werden können.

In einer weiteren bevorzugten Ausführungsform enthalten die erfindungsgemäßen Wirkstoffzubereitungen mindestens ein Vitamin, Pro-Vitamin, eine Vitaminvorstufe oder deren Derivate. Hierbei sind solche Komponenten bevorzugt, die üblicherweise den Vitamingruppen A, B, C, E, F und H zugeordnet werden.

Zur Gruppe der als Vitamin A bezeichneten Substanzen gehören das Retinol (Vitamin $A_1$) sowie das 3,4-Didehydroretinol (Vitamin $A_2$). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester, wie Retinylpalmitat und Retinylacetat in Betracht. Die erfindungsgemäßen Wirkstoffzubereitungen enthalten die Vitamin A-Komponente bevorzugt in Mengen von 0,05 - 1 Gew.-%, bezogen auf die gesamte flüssige Wirkstoffzubereitung.

Zur Vitamin B-Gruppe oder zu dem Vitamin B-Komplex gehören u. a.

- Vitamin $B_1$ (Thiamin)
- Vitamin $B_2$ (Riboflavin)
- Vitamin $B_3$. Unter dieser Bezeichnung werden häufig die Verbindungen Nicotinsäure und Nicotinsäureamid (Niacinamid) geführt. Erfindungsgemäß bevorzugt ist das Nicotinsäureamid, das bevorzugt in Mengen von 0,05 bis 1 Gew.-%, bezogen auf die gesamte flüssige Wirkstoffzubereitung, enthalten ist.
- Vitamin $B_5$ (Pantothensäure und Panthenol). Bevorzugt wird Panthenol eingesetzt. Erfindungsgemäß einsetzbare Derivate des Panthenols sind insbesondere die Ester und Ether des Panthenols sowie kationisch derivatisierte Panthenole. In einer weiteren bevorzugten Ausführungsform der Erfindung können an Stelle von sowie zusätzlich zu Pantothensäure oder Panthenol auch Derivate des 2-Furanon mit der allgemeinen Strukturformel (I) eingesetzt werden.

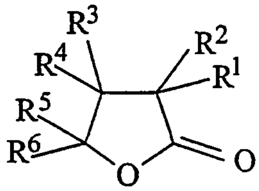

(I)

Bevorzugt sind die 2-Furanon-Derivate, in denen die Substituenten $R^1$ bis $R^6$ unabhängig voneinander ein Wasserstoffatom, einen Hydroxylrest, einen Methyl-, Methoxy-, Aminomethyl- oder Hydroxymethylrest, einen gesättigten oder ein- oder zweifach ungesättigten, linearen oder verzweigten $C_2$-$C_4$ - Kohlenwasserstoffrest, einen gesättigten oder ein- oder zweifach ungesättigten, verzweigten oder linearen Mono-, Di- oder Trihydroxy-$C_2$-$C_4$ - Kohlenwasserstoffrest oder einen gesättigten oder ein- oder zweifach ungesättigten, verzweigten oder linearen Mono-, Di- oder Triamino-$C_2$-$C_4$ - Kohlenwasserstoffrest darstellen. Besonders bevorzugte Derivate sind die auch im Handel erhältlichen Substanzen Dihydro-3-hydroxy-4,4-dimethyl-2(3H)-furanon mit dem Triviainamen Pantolacton (Merck), 4-Hydroxymethyl-γ-butyrolacton (Merck), 3,3-Dimethyl-2-hydroxy-γ-butyrolacton (Aldrich) und 2,5-Dihydro-5-methoxy-2-furanon (Merck), wobei ausdrücklich alle Stereoisomeren eingeschlossen sind. Das erfindungsgemäß außerordentlich bevorzugte 2-Furanon-Derivat ist Pantolacton (Dihydro-3-hydroxy-4,4-dimethyl-2(3H)-furanon), wobei in Formel (I) $R^1$ für eine Hydroxylgruppe, $R^2$ für ein Wasserstoffatom, $R^3$ und $R^4$ für eine Methylgruppe und $R^5$ und $R^6$ für ein Wasserstoffatom stehen. Das Stereoisomer (R)-Pantolacton entsteht beim Abbau von Pantothensäure. Die genannten Verbindungen des Vitamin $B_5$-Typs sowie die 2-Furanonderivate sind bevorzugt in einer Gesamtmenge von 0,05 bis 10 Gew.-%, besonders bevorzugt 0,1 bis 5 Gew.-%, jeweils bezogen auf die gesamte flüssige Wirkstoffzubereitung, enthalten.

- Vitamin $B_6$ (Pyridoxin sowie Pyridoxamin und Pyridoxal).
- Vitamin $B_7$ (Biotin), auch als Vitamin H oder "Hautvitamin" bezeichnet. Biotin ist bevorzugt in Mengen von 0,0001 bis 1,0 Gew.-%, insbesondere 0,001 bis 0,01 Gew.-%, jeweils bezogen auf die gesamte flüssige Wirkstoffzubereitung, enthalten.

Vitamin C (Ascorbinsäure) wird bevorzugt in Mengen von 0,1 bis 3 Gew.-%, bezogen auf die gesamte flüssige Wirkstoffzubereitung, eingesetzt. Die Verwendung der Derivate Ascorbylpalmitat, -stearat, -dipalmitat, -acetat, Mg-Ascorbylphosphat, Na-Ascorbylphosphat, Natrium- und Magnesiumascorbat, Dinatriumascorbylphosphat und -sulfat, Kaliumascorbyltocopherylphosphat, Chitosanascorbat oder Ascorbylglucosid kann bevorzugt sein. Die Verwendung in Kombination mit Tocopherolen kann ebenfalls bevorzugt sein.

Zur Vitamin E-Gruppe zählen Tocopherol, insbesondere $\alpha$-Tocopherol, und seine Derivate. Bevorzugte Derivate sind insbesondere die Ester, wie Tocopherylacetat,

-nicotinat, -phosphat, -succinat, -linoleat, -oleat, Tocophereth-5, Tocophereth-10, Tocophereth-12, Tocophereth-18, Tocophereth-50 und Tocophersolan.Tocopherol und seine Derivate sind bevorzugt in Mengen von 0,05 - 1 Gew.-%, bezogen auf die gesamte flüssige Wirkstoffzubereitung, enthalten.

Unter dem Begriff "Vitamin F" werden üblicherweise essentielle Fettsäuren, insbesondere Linolsäure, Linolensäure und Arachidonsäure, verstanden.

Bevorzugt enthalten die erfindungsgemäßen Mittel Vitamine, Provitamine und Vitaminvorstufen aus den Gruppen A, B, C, E und H. Vitamin A-palmitat (Retinylpalmitat), Panthenol und seine Derivate, Nicotinsäureamid, Ascorbylpalmitat, -acetat, Mg-Ascorbylphosphat, Na-Ascorbylphosphat, Natrium- und Magnesiumascorbat, die Tocopherolester, besonders Tocopherylacetat, und Biotin sind besonders bevorzugt.

In einer weiteren bevorzugten Ausführungsform enthalten die erfindungsgemäßen Wirkstoffzubereitungen mindestens einen Pflanzenextrakt als kosmetischen Wirkstoff. Die erfindungsgemäß geeigneten Pflanzenextrakte werden üblicherweise durch Extraktion der gesamten Pflanze, in einzelnen Fällen aber auch ausschließlich aus Blüten und/oder Blättern und/oder Samen und/oder anderen Pflanzenteilen hergestellt. Erfindungsgemäß bevorzugt sind vor allem die Extrakte aus dem Meristem, also dem teilungsfähigen Bildungsgewebe der Pflanzen, und speziellen Pflanzen wie Grünem Tee, Hamamelis, Kamille, Ringelblume, Stiefmütterchen, Paeonie, Aloe Vera, Rosskastanie, Salbei, Weidenrinde, Zimtbaum (cinnamon tree), Chrysanthemen, Eichenrinde, Brennessel, Hopfen, Klettenwurzel, Schachtelhalm, Weißdorn, Lindenblüten, Mandeln, Fichtennadeln, Sandelholz, Wacholder, Kokosnuß, Kiwi, Guave, Limette, Mango, Aprikose, Weizen, Melone, Orange, Grapefruit, Avocado, Rosmarin, Birke, Buchensprossen, Malve, Wiesenschaumkraut, Schafgarbe, Quendel, Thymian, Melisse, Hauhechel, Eibisch (Althaea), Malve (Malva sylvestris), Veilchen, Blättern der Schwarzen Johannisbeere, Huflattich, Fünffingerkraut, Ginseng, Ingwerwurzel, Süßkartoffel und Olive.

Vorteilhaft eingesetzt werden können auch Algenextrakte. Die erfindungsgemäß verwendeten Algenextrakte stammen aus Grünalgen, Braunalgen, Rotalgen oder Blaualgen (Cyanobakterien). Die zur Extraktion eingesetzten Algen können sowohl natürlichen Ursprungs als auch durch biotechnologische Prozesse gewonnen und gewünschtenfalls gegenüber der natürlichen Form verändert sein. Die Veränderung der Organismen kann gentechnisch, durch Züchtung oder durch die Kultivation in mit ausgewählten Nährstoffen angereicherten Medien erfolgen. Bevorzugte Algenextrakte stammen aus Seetang, Blaualgen, aus der Grünalge Codium tomentosum sowie aus der Braunalge Fucus vesiculosus. Ein besonders bevorzugter Algenextrakt stammt aus Blaualgen der Species Spirulina, die in einem Magnesium-angereicherten Medium kultiviert wurden.

Vorteilhaft eingesetzt werden können auch Extrakte aus Mikroorganismen, z. B. aus Hefen, bevorzugt aus Bäckerhefe.

Besonders bevorzugt sind die Extrakte aus Spirulina, Bäckerhefe, Grünem Tee, Aloe Vera, Meristem, Hamamelis, Aprikose, Ringelblume, Guave, Süßkartoffel, Limette, Mango, Kiwi, Gurke, Malve, Eibisch, Veilchen und Olive. Die erfindungsgemäßen Mittel können auch Mischungen aus mehreren, insbesondere aus zwei, verschiedenen Pflanzenextrakten enthalten.

Sebumregulierende Wirkstoffe werden im Sinne der Erfindung besonders bevorzugt ausgewählt aus wasser- und öllöslichen Extrakten aus Hamamelis, Klettenwurzel und Brennnessel, Zimtbaumextract (z. B. Sepicontrol® A5 von der Firma Seppic), Chrysanthemenextrakt (z. B. Laricyl® von Laboratoires Serobiologiques) und aus handelsüblichen Wirkstoffmischungen, z. B. Asebiol® BT 2 (INCI: Aqua, Hydrolyzed Yeast Protein, Pyridoxine, Niacinamide, Glycerin, Panthenol, Allantoin, Biotin) der Firma Laboratoires Serobiologiques und Antifettfaktor® COS-218/2-A (von Cosmetochem, INCI: Aqua, Cetyl-PCA, PEG-8 Isolauryl Thioether, PCA, Cetyl Alcohol).

Adstringierende Wirkstoffe werden im Sinne der Erfindung besonders bevorzugt ausgewählt aus wasser- und öllöslichen

Extrakten aus Hamamelis, Weidenrinde, Eichenrinde, Fünffingerkraut, Spitzwegerich und Salbei.

Da die erfindungsgemäß geeigneten Pflanzenextrakte Flavonoide enthalten, sind auch Flavonoide explizit als erfindungsgemäß geeignete kosmetische Wirkstoffe offenbart.

Die erfindungsgemäß bevorzugten Flavonoide umfassen die Glycoside der Flavone, der Flavanone, der 3-Hydroxyflavone (Flavonole), der Aurone und der Isoflavone. Besonders bevorzugte Flavonoide sind ausgewählt aus Naringin (Aurantiin, Naringenin-7-rhamnoglucosid), α-Glucosylrutin, α-Glucosylmyricetin, α-Glucosylisoquercetin, α-Glucosylquercetin, Hesperidin (3',5,7-Trihydroxy-4'-methoxyflavanon-7-rhamnoglucosid, Hesperitin-7-O-rhamnoglucosid), Neohesperidin, Rutin (3,3',4',5,7-Pentahydroxyflavon-3-rhamnoglucosid, Quercetin-3-rhamnoglucosid), Troxerutin (3,5-Dihydroxy- 3', 4', 7- tris (2- hydroxyethoxy)-flavon- 3-(6-0-(6- deoxy- α- L- mannopyranosyl)- β- D- glucopyranosid)), Monoxerutin (3,3',4',5-Tetrahydroxy-7-(2-hydroxyethoxy)-flavon-3-(6-O-(6-deoxy-α-L-rhannopyranosyl)-β-D-glucopyranosid)), Diosmin (3',4',7-Trihydroxy-5-methoxyflavanon-7-rhamnoglucosid) und Eriodictin.

Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phlorizin und Neohesperidindihydrochalkon.

Erfindungsgemäß werden die Flavonoide in Mengen von 0,001 bis 1 Gew.-%, bevorzugt 0,005 bis 0,5 Gew.-% und besonders bevorzugt 0,01 bis 0,4 Gew.-%, jeweils bezogen auf die Flavonoidaktivsubstanz in der gesamten flüssigen Wirkstoffzubereitung, eingesetzt.

Da die erfindungsgemäß geeigneten Pflanzenextrakte Polyphenole enthalten, sind auch Polyphenole explizit als erfindungsgemäß geeignete kosmetische Wirkstoffe offenbart.

Unter Polyphenolen sind erfindungsgemäß aromatische Verbindungen zu verstehen, die mindestens zwei phenolische Hydroxy-Gruppen im Molekül enthalten. Hierzu zählen die drei Dihydroxybenzole Brenzcatechin, Resorcin und Hydrochinon, weiterhin Phloroglucin, Pyrogallol und Hexahydroxybenzol. In der Natur treten freie und veretherte Polyphenole beispielsweise in Blütenfarbstoffen (Anthocyanidine, Flavone), in Gerbstoffen (Catechine, Tannine), als Flechten- oder Farn-Inhaltsstoffe (Usninsäure, Acylpolyphenole), in Ligninen und als Gallussäure-Derivate auf. Bevorzugte Polyphenole sind Flavone, Catechine, Usninsäure, und als Tannine die Derivate der Gallussäure, Digallussäure und Digalloylgallussäure. Besonders bevorzugte Polyphenole sind die monomeren Catechine, das heißt die Derivate der Flavan-3-ole, und Leukoanthocyanidine, das heißt die Derivate der Leukoanthocyanidine, die bevorzugt in 5,7,3',4',5'-Stellung phenolische Hydroxygruppen tragen, bevorzugt Epicatechin und Epigallocatechin, sowie die daraus durch Selbstkondensation entstehenden Gerbstoffe. Solche Gerbstoffe werden bevorzugt nicht in isolierter Reinsubstanz, sondern als Extrakte gerbstoffreicher Pflanzenteile eingesetzt, z. B. Extrakte von Catechu, Quebracho und Eichenrinde sowie anderer Baumrinden, Blättern von Grünem Tee (camellia sinensis) und Mate. Ebenfalls besonders bevorzugt sind die Tannine.

Erfindungsgemäß werden die Polyphenole in Mengen von 0,001 bis 10 Gew.-%, bevorzugt 0,005 bis 5 Gew.-% und besonders bevorzugt 0,01 bis 3 Gew.-%, jeweils bezogen auf die gesamte flüssige Wirkstoffzubereitung, eingesetzt.

Weiterhin hat es sich als besonders vorteilhaft erwiesen, dass die erfindungsgemäßen Wirkstoffzubereitungen mindestens ein Proteinhydrolysat oder dessen Derivat als kosmetischen Wirkstoff enthalten. Erfindungsgemäß können sowohl pflanzliche als auch tierische Proteinhydrolysate eingesetzt werden. Tierische Proteinhydrolysate sind z. B. Elastin-, Collagen-, Keratin-, Seiden- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Mandel-, Erbsen-, Kartoffel- und Reisproteinhydrolysate. Entsprechende Handelsprodukte sind z. B. DiaMin® (Diamalt), Gluadin® (Cognis), Lexein® (Inolex) und Crotein® (Croda).

Wenngleich der Einsatz der Proteinhydrolysate als solche bevorzugt ist, können an ihrer Stelle gegebenenfalls auch anderweitig erhaltene Aminosäuregemische oder einzelne Aminosäuren wie beispielsweise Arginin, Lysin, Histidin oder. Pyrroglutaminsäure eingesetzt werden. Ebenfalls möglich ist der Einsatz von Derivaten der Proteinhydrolysate, z. B. in Form ihrer Fettsäure-Kondensationsprodukte. Entsprechende Handelsprodukte sind z. B. Lamepon® (Cognis), Gluadin® (Cognis), Lexein® (Inolex), Crolastin® oder Crotein® (Croda).

Erfindungsgemäß einsetzbar sind auch kationisierte Proteinhydrolysate, wobei das zugrunde liegende Proteinhydrolysat vom Tier, von der Pflanze, von marinen Lebensformen oder von biotechnologisch gewonnenen Proteinhydrolysaten, stammen kann. Bevorzugt sind kationische Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Weiterhin können die kationischen Proteinhydrolysate auch noch weiter derivatisiert sein. Als typische Beispiele für erfindungsgemäß verwendete kationische Proteinhydrolysate und -derivate seien aufgeführt: Cocodimonium Hydroxypropyl Hydrolyzed Collagen, Cocodimonium Hydroxypropyl Hydrolyzed Casein, Steardimonium Hydroxypropyl Hydrolyzed Collagen.

In den erfindungsgemäßen Mitteln sind die Proteinhydrolysate und deren Derivate in Mengen von 0,01 - 10 Gew.-% bezogen auf die gesamte flüssige Wirkstoffzubereitung, enthalten. Mengen von 0,1 bis 5 Gew.%, insbesondere 0,1 bis 3 Gew.-%, sind besonders bevorzugt.

Aufgrund der einweißkoagulierenden Wirkung der Antitranspirant-Salze sollten diese nicht mit Proteinhydrolysaten kombiniert werden.

Besonders bevorzugte Komponenten mit kosmetischer und dermatologischer Wirkung sind außerdem Allantoin, Bisabolol, Pyrrolidoncarbonsäure (PCA) und ihre Ester mit Fettalkoholen sowie Isolaurylthioether und seine Derivate.

In einer weiteren bevorzugten Ausführungsform enthalten die erfindungsgemäßen Wirkstoffzubereitungen mindestens einen durchblutungsfördernden Wirkstoff und können damit als Anti-Cellulite-Mittel verwendet werden. Die durchblutungsfördernden Wirkstoffe sind ausgewählt aus Nicotinsäurederivaten mit vasodilatorischer Wirkung, Capsaicin, Extrakten aus Chilischoten (red pepper), Rutin und Rutinderivaten, Coffein und Rosskastanienextrakt sowie Mischungen hiervon. Ein erfindungsgemäß besonders bevorzugtes durchblutungsförderndes Nicotinsäurederivat ist das Vitamin E-nicotinat (Tocopherolnicotinat). Aufgrund ihrer verschieden starken Wirkung werden diese Stoffe in unterschiedlichen Gewichtsanteilen eingesetzt. Die bevorzugten Einsatzmengen betragen für Vitamin E-nicotinat 0,1 - 2,0 Gew.-% Reinsubstanz und für das Chiliextraktpulver 0,01 - 0,1 Gew.-%, bezogen auf die gesamte flüssige Wirkstoffzubereitung. Coffein, Rutin und Rosskastanienextrakt werden bevorzugt in Form wässrigalkoholischer Lösungen mit einem Aktivsubstanzgehalt von 0,1-10 Gew.-% eingesetzt. Der Anteil dieser handelsüblichen Lösungen an den erfindungsgemäßen flüssigen Wirkstoffzubereitungen beträgt 1 -10 Gew.-%.

In einer weiteren bevorzugten Ausführungsform enthalten die erfindungsgemäßen Wirkstoffzubereitungen mindestens einen hautaufhellenden Wirkstoff, ausgewählt aus den Derivaten der Ascorbinsäure, aus Kojisäure, Hydrochinon, Arbutin, Maulbeerbaumextrakt und Süßholzextrakt. Besonders bevorzugte Ascorbinsäurederivate sind Natriumascorbylphosphat, Magnesiumascorbylphosphat, Ascorbylmonopalmitat, Ascorbyldipalmitat, Ascorbylmonostearat, Ascorbyldistearat, Ascorbylmonoethylhexanoat, Ascorbyldiethylhexanoat, Ascorbylmonooctanoat, Ascorbyldioctanoat, Ascorbylmonoisostearat und Ascorbyldiisostearat.

Desweiteren lassen sich die erfindungsgemäßen treibgasfreien Sprayzubereitungen mit dermatologischen Wirkstoffen, z. B. Sebostatika, Anti-Akne-Wirkstoffen, Antiphlogistica oder Lokalanästhetika, formulieren.

Als verdickende Schichtsilikate können alle pulverförmigen Phyllosilikate, Magnesium-oder Alumosilikate eingesetzt werden, die aufgrund ihrer Blatt- oder Schichtstruktur in Wasser quellen und in dispergierter Form wäßrige Systeme verdicken und strukturviskos machen. Geeignete Schichtsilikate sind vor allem Smektite wie z. B. Talkum, Montmorillonit, Saponite oder Hectorite, sowie Smektite enthaltende Tone, z. B. Bentonite, weiterhin die unter dem Handelsnamen Veegum® von der Firma Vanderbilt vertriebenen Magnesiumaluminiumsilikate, die unter dem Handelsnamen Magnabrite® von der American Colloid Company vertriebenen Magnesiumaluminiumsilikate, die unter den Handelsnamen Hectabrite® und Hectalite® von der American Colloid Company vertriebenen Schichtsilikate (Hectorite) oder die unter dem Handelsnamen Bentone® von der Firma Rheox vertriebenen Schichtsilikate, z. B. Bentone® EW, ein komplexes Magnesiumlithiumsilikat. Ebenfalls geeignet sind die von Solvay unter der Bezeichnung Laponite® vertriebenen synthetischen Magnesiumsilikate, z. B. Laponite® XLS oder Laponite® XLG. Die Laponite® -Typen sind jedoch eher in schwach basischen bis neutralen pH-Bereichen stabil, so dass sie weniger geeignet sind zur Verdickung von Alminiumsalz-haltigen Zusammensetzungen. Die erfindungsgemäß geeigneten Schichtsilikate weisen Korngrößen von 0,5 bis 1000 μm, bevorzugt 1 bis 800 μm und besonders bevorzugt 10 bis 100 μm auf, jeweils bezogen auf die längste Teilchenausdehnung und gemessen mittels Siebanalyse an den trockenen Teilchen. In einer außerordentlich bevorzugten Ausführungsform ist als verdickendes Schichtsilikat ein Hectorit und/oder Bentonit mit einer Korngröße, bezogen auf die längste Teilchenausdehnung, von 74 μm (200 mesh) enthalten.

Die Einsatzmenge der verdickenden Schichtsilikate richtet sich nach deren Verdickungsleistung sowie nach den übrigen Zusatzstoffen der erfindungsgemäßen flüssigen Wirkstoffzubereitungen. Die Viskositäten der flüssigen Wirkstoffzubereitungen wurden mit einem Viskosimeter der Firma Haake, Modell Rheostress 150, im Kegel-Platte-System (60 mm/1°; 0,053 mm Spalt) bei Schergeschwindigkeiten von 0,06 - 100/s und einer Temperatur von 25°C gemessen. Die nach diesem Verfahren bestimmte Viskosität der erfindungsgemäßen flüssigen Wirkstoffzubereitungen beträgt 100 bis 10000 m-Pa·s, bevorzugt 200 bis 8000 m-Pa·s und besonders bevorzugt 300 bis 7000 m-Pa·s, jeweils bei einer geringen Schergeschwindigkeit von 1/s und 25 °C. Bei einer Schergeschwindigkeit von 100/s und mehr und 25 °C sollte die scheinbare Viskosität nicht mehr als 50 %, bevorzugt nicht mehr als 20 % und besonders bevorzugt nicht mehr als 5 % der Viskosität bei 1/s betragen. Daraus ergeben sich scheinbare Viskositäten der erfindungsgemäßen flüssigen Wirkstoffzubereitungen bei 100/s und 25 °C von maximal 50 bis 5000 m·Pa·s, bevorzugt maximal 20 bis 2000 m·Pa·s und besonders bevorzugt maximal 5 bis 500 m·Pa·s. Um diese Viskositäten auszubilden, ist in der Regel eine Menge von 0,2 - 2 Gew.-% des verdickenden Schichtsilikats, bezogen auf die gesamte flüssige Wirkstoffzubereitung, ausreichend. Dabei können auch Mischungen verschiedener Schichtsilikate eingesetzt werden.

Als Träger für die Wirkstoffe und verdickenden Schichtsilikate dienen feinteilige Öl-in-Wasser-Emulsionen oder Öl-in-Wasser-Mikroemulsionen mit einer Tröpfchengröße von weniger als 500 nm (Nanometer). Die Herstellung von geeigneten Emulsionssystemen ist literaturbekannt. So sind z.B. in DE 19514 269 A1 feinteilige Öl-in-Wasser-Emulsionen mit Tröpfchendurchmessem von 100-300 nm beschrieben, die adstringierende Antitranspirant-Wirkstoffe enthalten und durch ein Phaseninversions-Emulgierverfahren hergestellt werden. Aus DE 43 37041 A1 waren feinteilige Öl-in-Wasser-Emulsionen bekannt, die einen Deodorant-Wirkstoff enthalten und nach dem Phaseninversions-Emulgierverfahren zugänglich sind. Schließlich ist aus DE 195 30 220 A1 ein Verfahren zur Herstellung von transluzenten Öl-in-Wasser-Mikroemulsionen mit Teilchendurchmessern von weniger als 100 nm bekannt, die einen adstringierenden Antitranspirant-Wirkstoff enthalten. Aus den genannten Druckschriften kann man die für die Herstellung von feinteiligen

Öl-in-Wasser-Emulsionen geeigneten Ölkomponenten, Emulgatoren und Hilfsmittel entnehmen.

Die in den erfindungsgemäßen flüssigen Wirkstoffzubereitungen enthaltenen emulgierten Lipide umfassen sowohl die zur Bildung der feinteiligen Öl-in-Wasser-Emulsionen oder Öl-in-Wasser-Mikroemulsionen geeigneten Ölkomponenten als auch fettlösliche Wirkstoffe, z. B. fettlösliche Vitamine oder organische UV-Filter. Die emulgierten Lipide sind zu einem Anteil von 0,5 - 30 Gew.-%, bevorzugt 2-25 Gew.-% und besonders bevorzugt 4-20 Gew.-%, jeweils bezogen auf die Gesamtzusammensetzung, enthalten.

Als Ölkomponenten eignen sich alle wasserunlöslichen, hautverträglichen Öle und Fettstoffe und deren Gemische mit festen Paraffinen und Wachsen. Der Schmelzpunkt der gegebenenfalls verwendeten Gemische mit festen Paraffinen oder Wachsen sollte jedoch möglichst unterhalb der Phaseninversionstemperatur der Emulsion, bevorzugt unterhalb von 40 °C liegen. Außerdem sollte die Ölphase nicht zu unpolar sein.

Geeignete Ölkomponenten sind die Ester von gesättigten und ungesättigten, linearen und verzweigten $C_3$-$C_{22}$-Fettsäuren und den Dimeren ungesättigter $C_{12}$-$C_{22}$-Fettsäuren (Dimerfettsäuren) mit a) einwertigen linearen, verzweigten und cyclischen $C_2$-$C_{18}$-Alkanolen, b) mehrwertigen linearen und verzweigten $C_2$-$C_6$-Alkanolen, c) Polyglycerinen der Formel $CH_2OH$-$CHOH$-$CH_2[$-$O$-$CH_2$-$CHOH$-$CH_2]_n$-$O$-$CH_2$-$CHOH$-$CH_2OH$ mit n = 0 - 8, und d) den Dicarbonsäureestern von linearen und verzweigten $C_2$-$C_{10}$-Alkanolen, den Benzoesäureestern von linearen und verzweigten $C_{10}$-$C_{20}$-Alkanolen, den $C_{12}$-$C_{22}$-Alkanolestern ein- und mehrwertiger $C_2$-$C_7$-Hydroxycarbonsäuren, die aromatisch sein können, sowie Mischungen dieser Komponenten.

Beispiele für erfindungsgemäß besonders geeignete Fettsäureester einwertiger linearer oder verzweigter $C_2$-$C_{18}$-Alkanole (Typ a) sind Octylethylhexanoat, Isopropylpalmitat, Cetyloleat, 2-Hexyldecyllaurat (im Gemisch mit 2-Hexyldecanol als Handelsprodukt Cetiol® PGL erhältlich), Hexyllaurat, Isopropylmyristat, ein Gemisch der Ester von Caprylsäure und Caprinsäure mit $C_{12}$-$C_{18}$-Fettalkoholen, das als Handelsprodukt Cetiol® LC (Cognis) erhältlich ist, 2-Ethylhexylcaprylat, $C_{12}$-$C_{15}$-Alkyloctanoat, Isodecylneopentanoat, Isononylisononanoat, 2-Ethylhexylpalmitat (z. B. Cegesoft® C 24 von Cognis), 2-Ethylhexylstearat, Cetearylisononanoat (Mischung aus Cetylisononanoat und Stearylisononanoat, z. B. Cetiol® SN (Cognis)), Cetearyloctanoat, Ethylstearat, Isopropylstearat, Butylstearat und Myristylpropionat, außerdem natürlich vorkommende Esteröle wie z. B. Jojobaöl. Weiterhin geeignet sind die Monoester des Isosorbid (1,4:3,6-Dianhydro-D-glucit) mit $C_2$-$C_{24}$-Fettsäuren, z. B. das Isosorbidmonolaurat.

Beispiele für erfindungsgemäß besonders geeignete $C_8$-$C_{22}$-Fettsäureester mehrwertiger $C_2$-$C_6$-Alkanole (Typ b) sind als pflanzliche und tierische Öle vorkommende Fettsäuretriglycerid-Öle, z.B. Sonnenblumenöl, Sojaöl, Sesamöl, Haselnußöl, Olivenöl, Mandelöl, Rapsöl, Nachtkerzenöl (evening primrose oil), Distelöl (Safloröl), Avocadoöl sowie die flüssigen Anteile des Kokosöls oder des Rindertalgs. Erfindungsgemäß außerordentlich gut geeignet sind weiterhin synthetische Fettsäuretriglycerid-Öle. Besonders bevorzugt sind die Triglyceride von gesättigten $C_8$-$C_{10}$-Fettsäuren, wobei die Fettsäuren sowohl unverzweigt, wie z. B. bei den Handelsprodukten Myritol® 318 und Myritol® 331 (Cognis) oder Miglyol® 812 (Hüls), als auch verzweigt sein können, wie z. B. bei den Handelsprodukten Estol® GTEH 3609 (Uniqema) oder Myritol® GTEH (Cognis).

Weitere geeignete Ester dieses Typs sind die Fettsäureester des Ethylenglycols, z. B. Ethylenglycoldioleat, Ethylenglycoldüsotridecanoat, des 1,2-Propylenglycols, z. B. Propylenglycoldi-(2-ethylhexanoat), Propylenglycoldüsostearat oder Propylenglycoldipelargoat, des Butandiols, z. B. Butandioldüsostearat, des Neopentylglycols, z. B. Neopentylglycoldiheptanoat oder Neopentylglycoldicaprylat, des Trimethylolpropans und des Pentaerythrits. Beispiele für erfindungsgemäß bevorzugte Ölkomponenten sind die 1,2-Propylenglycoldiester von gesättigten, unverzweigten $C_8$-$C_{10}$-Fettsäuren, besonders bevorzugt die Handelsprodukte Myritol® PC und Miglyol® 840.

Beispiele für erfindungsgemäß besonders geeignete Dicarbonsäureester (Typ d) sind Diisopropylsebacat, Diisopropyladipat, Dioctylmalat, Dioctylmaleat, Dicaprylylmaleat, Dioctyladipat, Di-n-butyladipat, Diisooctylsuccinat, Di-(2-hexyldecyl)-succinat und Diisotridecylacelaat.

Beispiele für erfindungsgemäß besonders geeignete Benzoesäureester von linearen oder verzweigten $C_{10}$-$C_{20}$-Alkanolen sind unter dem Warenzeichen Finsolv® (Hersteller: Finetex) erhältlich, z. B. Finsolv® TN (Benzoesäure- C12-C15-alkylester), Finsolv® SB (Benzoesäureisostearylester) und Finsolv® BOD (Benzoesäureoctyldocecylester).

Als $C_8$-$C_{22}$-Fettalkoholester einwertiger oder mehrwertiger $C_2$-$C_7$-Hydroxycarbonsäuren sind die Ester der Glycolsäure, Milchsäure, Äpfelsäure, Weinsäure, Citronensäure und Salicylsäure erfindungsgemäß besonders geeignet. Solche Ester auf Basis von linearen $C_{14/15}$-Alkanolen und von in 2-Position verzweigten $C_{12/13}$-Alkanolen sind unter dem Warenzeichen Cosmacol® von der Fa. Nordmann, Rassmann GmbH & Co, Hamburg, zu beziehen. Besonders bevorzugt sind die Handelsprodukte Cosmacol® ESI, Cosmacol® EMI und Cosmacol® ETI.

Eine weitere besonders geeignete Gruppe von Ölkomponenten sind die bei 20 °C flüssigen, z. B. ungesättigten oder verzweigten, $C_8$-$C_{30}$-Feftalkohole, insbesondere die Guerbetalkohole, z. B. 2-Hexyldecyllaurat, 2-Hexyldecanol und 2-Octyldodecanol. Ebenfalls besonders geeignete Ölkomponenten sind die Di-n-alkylether wie z. B. Di-n-octylether, Di-(2-ethylhexyl)ether, Laurylmethylether oder Octylbutylether, sowie die Di-n-alkylcarbonate, z. B. Di-n-octylcarbonat.

Weitere bevorzugte Ölkomponenten sind die bei 20 °C noch flüssigen Kohlenwasserstoffe, z. B. Paraffinöle, Isoparaffine und synthetische Kohlenwasserstoffe, z. B. 1,3-Di-(2-ethyl-hexyl)-cyclohexan (Cetiol® S).

Als Emulgatoren enthalten die PIT-Emulsionen einen oder mehrere nichtionische Emulgatoren, bevorzugt aus

der Gruppe der nichtionischen Ethylenoxid-Addukte, oder eine Kombination aus einem hydrophilen, nichtionischen Emulgator mit einem HLB-Wert von bevorzugt 10-19 und einem lipophilen Coemulgator. Unter dem HLB-Wert soll dabei eine Größe verstanden werden, die aus der Struktur des Emulgators errechnet werden kann gemäß

$$HLB = \frac{100 - L}{5}$$

worin L der Gewichtsanteil (in %) der lipophilen Gruppen, z. B. der Fettalkyl- bzw. Fettacylgruppen im Emulgator ist.

Erfindungsgemäß verwendbare hydrophile nichtionische Emulgatoren sind beispielsweise

- Anlagerungsprodukte von 4 bis 40 Mol Ethylenoxid und/oder 0 bis 5 Mol Propylenoxid an lineare $C_8$-$C_{30}$-Fettalkohole, an $C_{12}$-$C_{22}$-Fettsäuren und an $C_8$-$C_{15}$-Alkylphenole,
- $C_{12}$-$C_{22}$-Fettsäuremono- und -diester von Anlagerungsprodukten von 1 bis 30 Mol Ethylenoxid an $C_3$-$C_6$-Polyole, insbesondere an Glycerin,
- Ethylenoxid- und Polyglycerin-Anlagerungsprodukte an Methylglucosid-Fettsäureester, Fettsäurealkanolamide und Fettsäureglucamide,
- $C_8$-$C_{22}$-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen, z. B. das im Handel erhältliche Produkt Montanov® 68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten $C_8$-$C_{22}$-Fettsäuren,
- Sterole (Sterine). Als Sterole wird eine Gruppe von Steroiden verstanden, die am C-Atom 3 des Steroid-Gerüstes eine Hydroxylgruppe tragen und sowohl aus tierischem Gewebe (Zoosterole) wie auch aus pflanzlichen Fetten (Phytosterole) isoliert werden. Beispiele für Zoosterole sind das Cholesterol und das Lanosterol. Beispiele geeigneter Phytosterole sind Beta-Sitosterol, Stigmasterol, Campesterol und Ergosterol. Auch aus Pilzen und Hefen werden Sterole, die sogenannten Mykosterole, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate, beispielsweise Polyglycerinpoly-12-hydroxystearat (Handelsprodukt Dehymuls® PGPH).

Bevorzugte hydrophile Emulgatoren sind die Ethylenoxid-Anlagerungsprodukte an lineare $C_{16}$-$C_{30}$-Fettalkohole oder an Partialester von $C_3$-$C_6$-Polyolen mit $C_{12}$-$C_{22}$-Fettsäuren. Es kann besonders bevorzugt sein, als hydrophile Emulgatoren Alkylpolyglycoside der Formel RO - $(Z)_x$ einzusetzen, in der R eine $C_8$-$C_{22}$-Alkyl- oder Alkenylgruppe, Z einen Monosaccharidrest, insbesondere Glucose, und dessen Oligomerisationsgrad x eine Zahl von 1,1 bis 5, insbesondere von 1,2 bis 1,4 darstellt.

Bei den lipophilen Coemulgatoren handelt es sich bevorzugt um lineare gesättigte $C_{16}$-$C_{22}$-Fettalkohole, um Partialester von $C_3$-$C_6$-Polyolen mit gesättigten $C_{14}$-$C_{22}$-Fettsäuren, um freie $C_{16}$-$C_{22}$-Fettsäuren, um Ethylenglycol- oder Propylenglycolmonofettsäureester, um Fettsäurealkanolamide aus $C_{12}$-$C_{18}$-Fettsäuren mit Mono- oder Dialkanolaminen mit 2-4 C-Atomen in der Alkanolgruppe oder um Glycerinmonofettalkoholether von $C_{12}$-$C_{22}$-Fettalkoholen. Besonders bevorzugte Coemulgatoren sind beispielsweise Cetylalkohol, Stearylalkohol, Arachidylalkohol, Behenylalkohol sowie Gemische dieser Alkohole, außerdem die Monoglyceride von Palmitin- und/oder Stearinsäure, die Sorbitanmono-und/oder -diester von Myristinsäure, Palmitinsäure, Stearinsäure oder von Mischungen dieser Fettsäuren, weiterhin die Monoester von Trimethylolpropan, Erythrit oder Pentaerythrit mit gesättigten $C_{14}$-$C_{22}$-Fettsäuren.

Die Emulgatoren sind in einer Menge von 0,2 - 10 Gew.-%, bevorzugt 0,5 - 8 Gew.-% und besonders bevorzugt 1-7 Gew.-%, jeweils bezogen auf die gesamte flüssige Wirkstoffzubereitung, enthalten.

Bevorzugt enthalten die erfindungsgemäßen flüssigen Wirkstoffzubereitungen zusätzlich 1-10 Gew.-% eines wasserlöslichen Polyols. Unter Wasserlöslichkeit versteht man erfindungsgemäß, dass sich wenigstens 5 Gew.-% des Alkohols bei 20°C klar lösen oder aber - im Falle langkettiger oder polymerer Alkohole - durch Erwärmen der Lösung auf 50 °C bis 60 °C in Lösung gebracht werden können. Hierzu zählen wasserlösliche Diole, Triole und höherwertige Alkohole, Polyglycerine, Polyethylenglycole sowie Mono-und Disaccharide. Unter den Diolen eignen sich $C_2$-$C_{12}$-Diole, insbesondere 1,2-Propylenglycol, Butylenglycole wie z.B. 1,2-Butylenglycol, 1,3-Butylenglycol und 1,4-Butylenglycol, Hexandiole wie z. B. 1,6-Hexandiol. Weiterhin bevorzugt geeignet sind Glycerin und Polyglycerine, insbesondere Diglycerin und Triglycerin, 1,2,6-Hexantriol, Erythrit, Trimethylolpropan, Sorbit oder Methylglucosid sowie die Polyethylenglycole (PEG) PEG-400, PEG-600, PEG-1000, PEG-1550, PEG-3000 und PEG-4000.
Erfindungsgemäß geeignete Monosaccharide sind z. B. Glucose, Fructose, Galactose, Arabinose, Ribose, Xylose, Lyxose, Allose, Altrose, Mannose, Gulose, Idose und Talose, die Desoxyzucker Fucose und Rhamnose sowie Aminozucker wie z. B. Glucosamin oder Galactosamin. Bevorzugt sind Glucose, Fructose, Galactose, Arabinose und Fucose; Glucose ist besonders bevorzugt.
Erfindungsgemäß geeignete Disaccharide sind aus zwei Monosaccharideinheiten zusammengesetzt, z. B. Saccharose oder Lactose. Ein besonders bevorzugtes Disaccharid ist Saccharose. Ebenfalls besonders bevorzugt ist die Verwendung von Honig, der überwiegend Glucose und Saccharose enthält.
Erfindungsgemäß kann sowohl ein Polyol als auch ein Gemisch mehrerer Polyole eingesetzt werden. Die Menge des Polyols oder des Polyol-Gemisches in den erfindungsgemäßen Zusammensetzungen beträgt 1 - 50 Gew.-% und bevorzugt 5-40 Gew.-%, bezogen auf die gesamte flüssige Wirkstoffzubereitung.
Niedere, flüchtige Alkohole sollten nur in geringen Mengen, bevorzugt aber gar nicht in den erfindungsgemäßen flüssigen Wirkstoffzubereitungen enthalten sein, so sollte z. B. der Gehalt an Ethanol oder Isopropanol einen Wert von 5 Gew.-% nicht überschreiten.

Neben den genannten Komponenten des Trägers, des Wirkstoffs und des verdickenden Schichtsilikats können die erfindungsgemäßen Sprayzubereitungen weitere, für solche Zusammensetzungen übliche Hilfsmittel enthalten. Solche weiteren Hilfsmittel sind z. B. Farbstoffe, Säuren, Alkalien oder Puffersalze zur Einstellung und Stabilisierung des pH-Wertes. Die Menge all dieser weiteren Hilfsmittel sollte bevorzugt nicht höher als etwa 15 Gew.-%, bezogen auf die gesamte flüssige Wirkstoffzubereitung, liegen.

Die nachfolgenden Beispiele sollen den Erfindungsgegenstand näher erläutem, ohne ihn hierauf zu beschränken.

## Beispiel

### 1. Herstellung der Wirkstoffzubereitungen gemäß Tabelle I

Die Schichtsilikat-Quellung wurde durch Einstreuen des Bentone EWCE-Pulvers unter Rühren (Mizerscheibe, 1000 upM) in warmes Wasser und anschließendem Rühren und Abkühlen auf 20 °C hergestellt.

Die Ölkomponenten (Cetiol OE, Cetiol PGL, Cetiol LC, Cetiol CC) und Emulgatoren (Emulgade SE, Emulgin B2, Mergital B10) wurden zusammen mit der 5 %igen Schichtsilikat-Quellung auf 95 °C erwärmt und 5 Minuten unter Rühren vermischt. Sodann wurde Wasser von 20 °C unter Rühren (Mizerscheibe, ca. 600 UpM) eingemischt und noch 10 Minuten gerührt. Zu dem noch warmen Ansatz wurde dann die 50 %ige wäßrige Aluminiumchlorhydrat-Lösung (Locron-L) und das Glycerin zugegeben und das Rühren noch ca. 30 Minuten fortgesetzt. In diese Phase können noch Duftstoffe und temperaturempfindliche Wirkstoffe eingearbeitet werden. Die Zusammensetzung der Zubereitungen ist der Tabelle zu entnehmen.

### 2. Anwendungstechnische Prüfung:

Die Zusammensetzungen wurden in durchsichtigen Glasflaschen (75 ml) mit Pumpzerstäuber-Aufsatz eingefüllt. Ein Sprühstoß (ca. 0,18 g) der waagerecht gegen eine im Abstand von 25 cm stehende vertikale Glasscheibe abgegeben wurde, erzeugte dort einen kreisrunden Flüssigkeitsfilm (∅ ca. 20 cm), dessen Verhalten (Tropfenbildung) an der Glasoberfläche beurteilt wurde. Das Ergebnis ist der Tabelle 1 zu entnehmen. Eine Zusammensetzung gemäß Beispiel 2, jedoch ohne Zusatz von Bentone EWCE, wurde zum Vergleich (5V) geprüft.

**Tabelle I**

| | 1 | 2 | 3 | 4 | 5V |
|---|---|---|---|---|---|
| Cetiol® LC | 5 | 5 | - | - | 5 |
| Cetiol® CC | 5 | 5 | - | - | 5 |
| Cetiol® OE | - | - | 10 | 10 | - |
| Cetiol® PGL | - | - | 5 | 5 | - |
| Emulgade® SE | 4,1 | 4,1 | - | - | 4,1 |
| Eumulgin® B2 | 0,9 | 0,9 | - | - | 0,9 |
| Mergital® B10 | - | - | 3,75 | 3,75 | - |
| Bentone® EWCE (5 %ige Dispersion in Wasser) | 15 | 10 | 15 | 10 | - |
| Glycerin | 5 | 5 | 5 | 5 | 5 |
| Locron® -L (50%ig) | 40 | 40 | 40 | 40 | 40 |

(fortgesetzt)

| | 1 | 2 | 3 | 4 | 5V |
|---|---|---|---|---|---|
| Parfümöl<br>Wasser | 1,0<br>24,0 | 1,0<br>29,0 | 1,0<br>20,25 | 1,0<br>25,25 | 1,0<br>39,0 |
| Viskosität (mPas, 25 °C) 1/s<br>Viskosität (mPas, 25 °C) 100/s | 920<br>97 | 150<br>30 | 9937<br>160 | 2000<br>68 | <10<br><10 |
| Tropfenbildung an der vertikalen Glasoberfläche | keine | gering | keine | keine | stark |

**Tabelle II**

| | 6 | 7 Hautpflege | 8 Sonnenschutzmittel |
|---|---|---|---|
| Cetiol® OE | 10 | 10 | 10 |
| Cetiol® PGL | 5 | 5 | 5 |
| Tocopherylacetat | - | 0,5 | 0,5 |
| Uvinul® MS 40 | - | - | 2,0 |
| Mergital® B10 | 3,75 | 3,75 | 3,75 |
| Bentone® EWCE (5 %ige Dispersion in Wasser) | 40 | 20 | 20 |
| 1,2-Propylenglycol | 5,0 | 5,0 | 5,0 |
| Locron® -L (50%ig) | 10 | - | - |
| Parfümöl | 1,0 | 1,0 | 1,0 |
| Wasser | 25,25 | 54,75 | 52,75 |
| Tropfenbildung an der vertikalen Glasoberfläche | keine | keine | keine |

Es wurden folgende Handelsprodukte verwendet:

| | |
|---|---|
| Cetiol® LC: *(Cognis)* | Caprylsäure-Caprinsäure-$C_{12}$-$C_{18}$-Fettalkoholester INCI: Coco-Caprylate/Caprate |
| Cetiol® CC: *(Cognis)* | Dloctylcarbonat |
| Cetiol® OE: *(Cognis)* | Dioctylether |
| Cetiol® PGL: *(Cognis)* | 2-Hexyldecyllaurat, 2-Hexyldecanol (OHZ: 105) |
| Emulgade® SE: *(Cognis)* | Gemisch aus Glycerylstearat, Ceteareth 20, Ceteareth 12, Cetearylalcohol und Cetylpalmitat |
| Eumulgin® B2: *(Cognis)* | Ceteareth 20 (Cetomacrogol 1000) |
| Mergitah® B10: *(Cognis)* | geheneth 10 |
| Bentone® EWCE: *(Rheox)* | Hektorit, Pulver (Dichte 2,5 g/$cm^3$) |
| Locron® -L: *(Clariant)* | Aluminiumchlorhydrat, (50%ig in $H_2O$). |
| Uvinul® MS 40: *(BASF)* | 2-Hydroxy-4-methoxybenzophenon-5-sulfonsäure |

**Patentansprüche**

1. Treibgasfreie Sprayzubereitung zur topischen Applikation am menschlichen Körper, bestehend aus einem Spendebehälter mit Sprühapplikator und einer flüssigen Wirkstoffzubereitung in Form einer feinteiligen, strukturviskosen Öl-in-Wasser-Emulsion oder Öl-in-Wasser-Mikroemulsion mit einer Tröpfchengröße unter 500 nm, enthaltend

(A) eine feinteilige Öl-in-Wasser-Emulsion oder Öl-in-Wasser-Mikroemulsion mit einem Anteil von 0,5 - 30 Gew.-% emulgierter Lipide und 0,2 - 10 Gew.-% Emulgatoren, jeweils bezogen auf die gesamte flüssige Wirkstoffzubereitung,
(B) 1-30 Gew.%, bezogen auf die gesamte flüssige Wirkstoffzubereitung, wenigstens eines kosmetischen oder dermatologischen Wirkstoffs,
(C) 0,2-2 Gew.-%, bezogen auf die gesamte flüssige Wirkstoffzubereitung, eines verdickenden Schichtsilikats,

sowie gegebenenfalls bis zu 15 Gew.-%, bezogen auf die gesamte flüssige Wirkstoffzubereitung, weiterer Hilfsmittel.

**2.** Sprayzubereitungen gemäß Anspruch 1, **dadurch gekennzeichnet, daß** als kosmetischer Wirkstoff (B) mindestens ein wasserlösliches Salz des Aluminiums, Zirkoniums oder Zinks oder eine beliebige Mischung dieser Salze enthalten ist.

**3.** Sprayzubereitungen gemäß Anspruch 1 oder 2, **dadurch gekennzeichnet, daß** als kosmetischer Wirkstoff (B) mindestens ein Deodorans, ausgewählt aus Duftstoffen, antimikrobiellen, antibakteriellen, keimhemmenden oder enzymhemmenden Stoffen, Antioxidantien oder Geruchsadsorbentien, enthalten ist.

**4.** Sprayzubereitungen gemäß einem der vorhergehenden Ansprüche, **dadurch gekennzeichnet, daß** als kosmetischer Wirkstoff (B) mindestens eine organische und/oder anorganische UV-Filtersubstanz enthalten ist.

**5.** Sprayzubereitungen gemäß einem der vorhergehenden Ansprüche, **dadurch gekennzeichnet, daß** als kosmetischer Wirkstoff (B) mindestens ein insektenabweisender Wirkstoff enthalten ist.

**6.** Sprayzubereitungen gemäß einem der vorhergehenden Ansprüche, **dadurch gekennzeichnet, daß** als kosmetischer Wirkstoff (B) mindestens ein Vitamin, Pro-Vitamin, eine Vitaminvorstufe oder deren Derivat, zugeordnet den Vitamingruppen A, B, C, E, F und H, enthalten ist.

**7.** Sprayzubereitungen gemäß einem der vorhergehenden Ansprüche, **dadurch gekennzeichnet, daß** als kosmetischer Wirkstoff (B) mindestens ein Pflanzenextrakt, ein Algenextrakt, ein Extrakt aus Mikroorganismen, ein Flavonoid oder ein Polyphenol enthalten ist.

**8.** Sprayzubereitungen gemäß einem der vorhergehenden Ansprüche, **dadurch gekennzeichnet, daß** als kosmetischer Wirkstoff (B) mindestens ein Proteinhydrolysat oder dessen Derivat enthalten ist.

**9.** Sprayzubereitungen gemäß einem der vorhergehenden Ansprüche, **dadurch gekennzeichnet, daß** als kosmetischer Wirkstoff (B) mindestens ein durchblutungsfördernder Wirkstoff enthalten ist.

**10.** Sprayzubereitungen gemäß einem der vorhergehenden Ansprüche, **dadurch gekennzeichnet, daß** als kosmetischer Wirkstoff (B) mindestens ein hautaufhellender Wirkstoff enthalten ist.

**11.** Sprayzubereitungen gemäß einem der vorhergehenden Ansprüche, **dadurch gekennzeichnet, daß** als dermatologischer Wirkstoff (B) mindestens ein Sebostatikum, Anti-Akne-Wirkstoff, Antiphlogisticum oder Lokalanästhetikum enthalten ist.

**12.** Sprayzubereitungen gemäß einem der vorhergehenden Ansprüche, **dadurch gekennzeichnet, daß** das verdikkende Schichtsilikat (C) ausgewählt ist aus Hectoriten und Bentoniten.

**13.** Sprayzubereitungen gemäß einem der vorhergehenden Ansprüche, **dadurch gekennzeichnet, daß** das verdikkende Schichtsilikat (C) Korngrößen von 0,5 bis 1000 $\mu$m, jeweils bezogen auf die längste Teilchenausdehnung und gemessen mittels Siebanalyse an den trockenen Teilchen, aufweist.

**14.** Sprayzubereitungen gemäß einem der vorhergehenden Ansprüche, **dadurch gekennzeichnet, daß** die flüssige Wirkstoffzubereitung scheinbare Viskositäten bei einer Schergeschwindigkeit von 1/s und 25 °C von 100 bis 10000 m·Pa·s, und bei einer Schergeschwindigkeit von 100/s und 25 °C maximal 50 % der Viskosität bei 1/s aufweist, jeweils gemessen mit einem Viskosimeter der Firma Haake, Modell Rheostress 150, im Kegel-Platte-System (60 mm/1°; 0,053 mm Spalt).

**15.** Sprayzubereitungen gemäß einem der vorhergehenden Ansprüche, **dadurch gekennzeichnet, daß** die flüssige

Wirkstoffzubereitung scheinbare Viskositäten bei einer Schergeschwindigkeit von 1/s und 25 °C von 100 bis 10000 m·Pa·s, und bei einer Schergeschwindigkeit von 100/s und 25 °C maximal 20 % der Viskosität bei 1/s aufweist, jeweils gemessen mit einem Viskosimeter der Firma Haake, Modell Rheostress 150, im Kegel-Platte-System (60 mm/1°; 0,053 mm Spalt).

**16.** Sprayzubereitungen gemäß einem der vorhergehenden Ansprüche, **dadurch gekennzeichnet, daß** die flüssige Wirkstoffzubereitung scheinbare Viskositäten bei einer Schergeschwindigkeit von 1/s und 25 °C von 100 bis 10000 m·Pa·s, und bei einer Schergeschwindigkeit von 100/s und 25 °C maximal 5 % der Viskosität bei 1/s aufweist, jeweils gemessen mit einem Viskosimeter der Firma Haake, Modell Rheostress 150, im Kegel-Platte-System (60 mm/1°; 0,053 mm Spalt).

**17.** Sprayzubereitungen gemäß einem der vorhergehenden Ansprüche, **dadurch gekennzeichnet, daß** das emulgierte Lipid ausgewählt ist aus flüssigen Triglyceriden, flüssigen Di-n-alkylethern, flüssigen Di-n-alkylcarbonaten, flüssigen, gegebenenfalls verzweigten $C_8$-$C_{30}$-Fettalkoholen, den Estern von gegebenenfalls verzweigten $C_8$-$C_{30}$-Fettalkoholen mit einer $C_8$-$C_{30}$-Fettsäure, sowie beliebigen Mischungen der genannten Lipide, wobei die genannten Komponenten jeweils bei 20 °C flüssig sind.

**18.** Verwendung einer Sprayzubereitung gemäß einem der Ansprüche 1, 4 sowie 6 bis 9 als Antifalten-Hautbehandlungsmittel oder als Antiage-Hautbehandlungsmittel.

**19.** Verfahren zur Herstellung einer treibgasfreien Sprayzubereitung zur topischen Applikation am menschlichen Körper, die aus einem Spendebehälter mit Sprühapplikator und einer flüssigen Wirkstoffzubereitung in Form einer feinteiligen, strukturviskosen Öl-in-Wasser-Emulsion oder Öl-in-Wasser-Mikroemulsion mit einer Tröpfchengröße unter 500 nm besteht, wobei eine feinteilige Öl-in-Wasser-Emulsion oder Öl-in-Wasser-Mikroemulsion mit einem Anteil von 0,5 - 30 Gew.-% emulgierter Lipide, 0,2 - 10 Gew.-% Emulgatoren, 1 - 30 Gew.-% wenigstens eines kosmetischen oder dermatologischen Wirkstoffs, 0,2 - 2 Gew.-% eines verdickenden Schichtsilikats, gegebenenfalls bis zu 15 Gew.-% weiterer Hilfsmittel, jeweils bezogen auf die gesamte flüssige Wirkstoffzubereitung, hergestellt und in einen treibgasfreien Spendebehälter mit Sprühapplikator eingefüllt wird.

**20.** Verwendung von mindestens einem verdickenden Schichtsilikat zur Verbesserung der Applikationseigenschaften einer treibgasfreien Sprayzubereitung zur topischen Applikation am menschlichen Körper, die aus einem Spendebehälter mit Sprühapplikator und einer flüssigen Wirkstoffzubereitung in Form einer feinteiligen, strukturviskosen Öl-in-Wasser-Emulsion oder Öl-in-Wasser Mikroemulsion mit einer Tröpfchengröße unter 500 nm besteht und eine feinteilige Öl-in-Wasser-Emulsion oder Öl-in-Wasser-Mikroemulsion mit einem Anteil von 0,5 - 30 Gew.-% emulgierter Lipide, 0,2 - 10 Gew.-% Emulgatoren, 1 - 30 Gew.-% wenigstens eines kosmetischen oder dermatologischen Wirkstoffs, 0,2 - 2 Gew.-% eines verdickenden Schichtsilikats sowie gegebenenfalls bis zu 15 Gew.-% weiterer Hilfsmittel, jeweils bezogen auf die gesamte flüssige Wirkstoffzubereitung, enthält.

## Claims

**1.** Propellant-gas-free spray preparation for topical application to the human body, consisting of a dispensing container with spray applicator and a liquid active ingredient preparation in the form of a finely divided, structurally viscous oil-in-water emulsion or oil-in-water microemulsion with a droplet size below 500 nm, comprising

(A) a finely divided oil-in-water emulsion or oil-in-water microemulsion with a content of 0.5-30% by weight of emulsified lipids and 0.2-10% by weight of emulsifiers, in each case based on the total liquid active ingredient preparation,
(B) 1-30% by weight, based on the total liquid active ingredient preparation, of at least one cosmetic or dermatological active ingredient,
(C) 0.2-2% by weight, based on the total liquid active ingredient preparation, of a thickening sheet silicate,

and optionally up to 15% by weight, based on the total liquid active ingredient preparation, of further auxiliaries.

**2.** Spray preparations according to Claim 1,
**characterized in that**, as cosmetic active ingredient (B), at least one water-soluble salt of aluminium, zirconium or zinc or any mixture of these salts is present.

**3.** Spray preparations according to Claim 1 or 2, **characterized in that**, as cosmetic active ingredient (B), at least one deodorant chosen from fragrances, antimicrobial, antibacterial, germ-inhibiting or enzyme-inhibiting substances, antioxidants or odour adsorbents is present.

**4.** Spray preparations according to one of the preceding claims, **characterized in that**, as cosmetic active ingredient (B), at least one organic and/or inorganic UV filter substance is present.

**5.** Spray preparations according to one of the preceding claims, **characterized in that**, as cosmetic active ingredient (B), at least one insect-repelling active ingredient is present.

**6.** Spray preparations according to one of the preceding claims, **characterized in that**, as cosmetic active ingredient (B), at least one vitamin, provitamin, vitamin precursor or derivative thereof, assigned to the vitamin groups A, B, C, E, F and H is present.

**7.** Spray preparations according to one of the preceding claims, **characterized in that**, as cosmetic active ingredient (B), at least one plant extract, one algae extract, an extract from microorganisms, a flavonoid or a polyphenol is present.

**8.** Spray preparations according to one of the preceding claims, **characterized in that**, as cosmetic active ingredient (B), at least one protein hydrolysate or derivative thereof is present.

**9.** Spray preparations according to one of the preceding claims, **characterized in that**, as cosmetic active ingredient (B), at least one circulation-promoting active ingredient is present.

**10.** Spray preparations according to one of the preceding claims, **characterized in that**, as cosmetic active ingredient (B), at least one skin-lightening active ingredient is present.

**11.** Spray preparations according to one of the preceding claims, **characterized in that**, as dermatogical active ingredient (B), at least one sebostatic, antiacne active ingredient, anti-phlogistic or local anaesthetic is present.

**12.** Spray preparations according to one of the preceding claims, **characterized in that** the thickening sheet silicate (C) is chosen from hectorites and bentonites.

**13.** Spray preparations according to one of the preceding claims, **characterized in that** the thickening sheet silicate (C) has particle sizes of from 0.5 to 1000 μm, in each case based on the longest particle extension and measured by means of sieve analysis on the dry particles.

**14.** Spray preparations according to one of the preceding claims, **characterized in that** the liquid active ingredient preparation has apparent viscosities at a shear rate of 1/s and 25°C of from 100 to 10 000 m.Pa.s, and at a shear rate of 100/s and 25°C of at most 50% of the viscosity at 1/s, in each case measured using a viscometer from Haake, Rheostress model 150, in the cone-plate system (60 mm/1°; 0.053 mm gap).

**15.** Spray preparations according to one of the preceding claims, **characterized in that** the liquid active ingredient preparation has apparent viscosities at a shear rate of 1/s and 25°C of from 100 to 10 000 m.Pa.s, and at a shear rate of 100/s and 25°C of at most 20% of the viscosity at 1/s, in each case measured using a viscometer from Haake, Rheostress model 150, in the cone-plate system (60 mm/1°; 0.053 mm gap).

**16.** Spray preparations according to one of the preceding claims, **characterized in that** the liquid active ingredient preparation has apparent viscosities at a shear rate of 1/s and 25°C of from 100 to 10 000 m.Pa.s, and at a shear rate of 100/s and 25°C of at most 5% of the viscosity at 1/s, in each case measured using a viscometer from Haake, Rheostress model 150, in the cone-plate system (60 mm/1°; 0.053 mm gap).

**17.** Spray preparations according to one of the preceding claims, **characterized in that** the emulsified lipid is chosen from liquid triglycerides, liquid di-n-alkyl ethers, liquid di-n-alkyl carbonates, liquid, optionally branched, $C_8$-$C_{30}$-fatty alcohols, the esters of optionally branched $C_8$-$C_{30}$-fatty alcohols with a $C_8$-$C_{30}$-fatty acid, and any mixtures of said lipids, where the specified components are in each case liquid at 20°C.

**18.** The use of a spray preparation according to one of Claims 1, 4 and 6 to 9 as antiwrinkle skin-treatment composition

or as antiageing skin-treatment composition.

**19.** Process for the preparation of a propellant-gas-free spray preparation for topical application to the human body which consists of a dispensing container with spray applicator and a liquid active ingredient preparation in the form of a finely divided, structurally viscous oil-in-water emulsion or oil-in-water microemulsion with a droplet size below 500 nm, where a finely divided oil-in-water emulsion or oil-in-water microemulsion with a content of 0.5-30% by weight of emulsified lipids, 0.2-10% by weight of emulsifiers, 1-30% by weight of at least one cosmetic or dermatological active ingredient, 0.2-2% by weight of a thickening sheet silicate, optionally up to 15% by weight of further auxiliaries, in each case based on the total liquid active ingredient preparation, is prepared and introduced into a propellant-gas-free dispensing container with spray applicator.

**20.** The use of at least one thickening sheet silicate for improving the application properties of a propellant-gas-free spray preparation for topical application to the human body which consists of a dispensing container with spray applicator and a liquid active ingredient preparation in the form of a finely divided, structurally viscous oil-in-water emulsion or oil-in-water microemulsion with a droplet size below 500 nm and comprises a finely divided oil-in-water emulsion or oil-in-water microemulsion with a content of 0.5-30% by weight of emulsified lipids, 0.2-10% by weight of emulsifiers, 1-30% by weight of at least one cosmetic or dermatological active ingredient, 0.2-2% by weight of a thickening sheet silicate and optionally up to 15% by weight of further auxiliaries, in each case based on the total liquid active ingredient preparation.

## Revendications

**1.** Préparation pour pulvérisation exempte de gaz propulseur destinée à l'application topique sur le corps humain, constituée par un récipient distributeur avec un dispositif d'application par pulvérisation et une préparation liquide de substance active sous forme d'une émulsion huile-dans-eau ou d'une micro-émulsion huile-dans-eau finement divisée, présentant une viscosité de structure, présentant une grosseur des gouttes inférieure à 500 nm, contenant

(A) une émulsion huile-dans-eau ou une micro-émutsion huile-dans-eau finement divisée présentant une proportion de 0,5 à 30% en poids de lipides émulsionnés et 0,2 à 10% en poids d'émulsifiants, à chaque fois par rapport à la préparation de substance active liquide totale,
(B) 1 à 30% en poids, par rapport à la préparation de substance active liquide totale d'au moins une substance active cosmétique ou dermatologique,
(C) 0,2 à 2% en poids, par rapport à la préparation de substance active liquide totale, d'un phyllosilicate à effet épaississant,

ainsi que le cas échéant jusqu'à 15% en poids, par rapport à la préparation de substance active liquide totale, d'autres adjuvants.

**2.** Préparations pour pulvérisation selon la revendication 1, **caractérisées en ce qu'**elles contiennent comme substance active cosmétique (B) au moins un sel soluble dans l'eau de l'aluminium, du zirconium ou du zinc ou un mélange quelconque de ces sels.

**3.** Préparations pour pulvérisation selon la revendication 1 ou 2, **caractérisées en ce qu'**elles contiennent comme substance active cosmétique (B) au moins un déodorant, choisi parmi les parfums, les substances antimicrobiennes, antibactériennes, inhibant les germes ou les enzymes, les antioxydants ou les agents d'adsorption des odeurs.

**4.** Préparations pour pulvérisation selon l'une quelconque des revendications précédentes, **caractérisées en ce qu'**elles contiennent comme substance active cosmétique (B) au moins une substance filtre des UV organique et/ou inorganique.

**5.** Préparations pour pulvérisation selon l'une quelconque des revendications précédentes, **caractérisées en ce qu'**elles contiennent comme substance active cosmétique (B) au moins une substance active de répulsion des insectes.

**6.** Préparations pour pulvérisation selon l'une quelconque des revendications précédentes, **caractérisées en ce qu'**elles contiennent comme substance active cosmétique (B) au moins une vitamine, une pro-vitamine, un précurseur de vitamine ou leurs dérivés, associés aux groupes de vitamines A, B, C, E, F et H.

**7.** Préparations pour pulvérisation selon l'une quelconque des revendications précédentes, **caractérisées en ce qu'**elles contiennent comme substance active cosmétique (B) au moins un extrait végétal, un extrait d'algues, un extrait de micro-organismes, un flavonoïde ou un polyphénol.

**8.** Préparations pour pulvérisation selon l'une quelconque des revendications précédentes, **caractérisées en ce qu'**elles contiennent comme substance active cosmétique (B) au moins un hydrolysat de protéines ou son dérivé.

**9.** Préparations pour pulvérisation selon l'une quelconque des revendications précédentes, **caractérisées en ce qu'**elles contiennent comme substance active cosmétique (B) au moins une substance active favorisant la circulation sanguine.

**10.** Préparations pour pulvérisation selon l'une quelconque des revendications précédentes, **caractérisées en ce qu'**elles contiennent comme substance active cosmétique (B) au moins une substance active éclaircissant la peau.

**11.** Préparations pour pulvérisation selon l'une quelconque des revendications précédentes, **caractérisées en ce qu'**elle contiennent comme substance active dermatologique (B) au moins un sébostatique, une substance active anti-acnée, un antiphlogistique ou un anesthésique local.

**12.** Préparations pour pulvérisation selon l'une quelconque des revendications précédentes, **caractérisées en ce que** le phyllosilicate à effet épaississant (C) est choisi parmi les hectorites et les bentonites.

**13.** Préparations pour pulvérisation selon l'une quelconque des revendications précédentes, **caractérisées en ce que** le phyllosilicate à effet épaississant (C) présente des granulométries de 0,5 à 1000 $\mu$m, à chaque fois par rapport à l'étendue la plus longue des particules et mesurées par analyse granulométrique sur les particules sèches.

**14.** Préparations pour pulvérisation selon l'une quelconque des revendications précédentes, **caractérisées en ce que** la préparation de substance active liquide présente des viscosités apparentes à une vitesse de cisaillement de 1/s et à 25°C de 100 à 10000 mPa.s, et à une vitesse de cisaillement de 100/s et à 25°C d'au maximum 50% de la viscosité à 1/s, à chaque fois mesurées au moyen d'un viscosimètre de la société Haake, modèle Rheostress 150, dans un système cône-plaque (60 mm/1 °; fente de 0,053 mm).

**15.** Préparations pour pulvérisation selon l'une quelconque des revendications précédentes, **caractérisées en ce que** la préparation de substance active liquide présente des viscosités apparentes à une vitesse de cisaillement de 1/s et à 25°C de 100 à 10000 mPa.s, et à une vitesse de cisaillement de 100/s et à 25°C d'au maximum 20% de la viscosité à 1/s, à chaque fois mesurées au moyen d'un viscosimètre de la société Haake, modèle Rheostress 150, dans le système cône-plaque (60 mm/1°; fente de 0,053 mm).

**16.** Préparations pour pulvérisation selon l'une quelconque des revendications précédentes, **caractérisées en ce que** la préparation de substance active liquide présente des viscosités apparentes à une vitesse de cisaillement de 1/s et à 25°C de 100 à 10000 mPa.s, et à une vitesse de cisaillement de 100/s et à 25°C d'au maximum 5% de la viscosité à 1/s, à chaque fois mesurées sur un viscosimètre de la société Haake, modèle Rheostress 150, dans le système cône-plaque (60 mm/1°; fente de 0,053 mm).

**17.** Préparations pour pulvérisation selon l'une quelconque des revendications précédentes, **caractérisées en ce que** le lipide émulsionné est choisi parmi les triglycérides liquides, les di-n-alkyléthers liquides, les di-n-alkylcarbonates liquides, les alcools gras en $C_8$ à $C_{30}$, le cas échéant ramifiés, liquides, les esters d'alcools gras en $C_8$ à $C_{30}$ le cas échéant ramifiés avec un acide gras en $C_8$ à $C_{30}$ ainsi que les mélanges quelconques des lipides mentionnés, les composants mentionnés étant à chaque fois liquides à 20°C.

**18.** Utilisation d'une préparation pour pulvérisation selon l'une quelconque des revendications 1, 4 ainsi que 6 à 9 comme agent de traitement antiride pour la peau ou comme agent de traitement antivieillissement de la peau.

**19.** Procédé pour la fabrication d'une préparation pour pulvérisation exempte de gaz propulseur destinée à l'application topique sur le corps humain, qui est constituée par un récipient distributeur avec un dispositif d'application par pulvérisation et une préparation de substance active liquide sous forme d'une émulsion huile-dans-eau ou d'une micro-émulsion huile-dans-eau finement divisée à viscosité de structure présentant une grosseur des gouttes inférieure à 500 nm, où on prépare une émulsion huile-dans-eau ou une micro-émulsion huile-dans-eau finement divisée présentant une proportion de 0,5 à 30% en poids de lipides émulsionnés, 0,2 à 10% en poids d'émulsifiants, 1 à

30% en poids d'au moins une substance active cosmétique ou dermatologique, 0,2 à 2% en poids d'un phyllosilicate à effet épaississant, le cas échéant jusqu'à 15% en poids d'autres adjuvants, à chaque fois par rapport à la préparation de substance active liquide totale, et on la conditionne dans un récipient distributeur exempt de gaz propulseur avec un dispositif d'application par pulvérisation.

**20.** Utilisation d'au moins un phyllosilicate à effet épaississant pour améliorer les propriétés d'application d'une préparation pour pulvérisation exempte de gaz propulseur destinée à une application topique sur le corps humain, qui est constituée par un récipient distributeur avec un dispositif d'application par pulvérisation et une préparation de substance active liquide sous forme d'une émulsion huile-dans-eau ou d'une micro-émulsion huile-dans-eau finement divisée à viscosité de structure présentant une grosseur des gouttes inférieure à 500 nm, et qui contient une émulsion huile-dans-eau ou une micro-émulsion huile-dans-eau finement divisée présentant une proportion de 0,5 à 30% en poids de lipides émulsionnés, 0,2 à 10% en poids d'émulsifiants, 1 à 30% en poids d'au moins une substance active cosmétique ou dermatologique, 0,2 à 2% en poids d'un phyllosilicate à effet épaississant, ainsi que, le cas échéant, jusqu'à 15% en poids d'autres adjuvants, à chaque fois par rapport à la préparation de substance active liquide totale.